# EUROPEAN PATENT SPECIFICATION

(11) **EP 4 185 304 B1**
(45) Date of publication and mention of the grant of the patent: **22.04.2026**
(21) Application number: 21845276.1
(22) Date of filing: 22.07.2021
(51) Int. Cl.: A61K 40/11, A61K 40/31, A61K 40/42, A61K 9/00, A61K 9/06, A61K 9/19, A61K 9/51, A61P 35/00

(54) **THERAPEUTIC HYDROGEL FOR THE DELIVERY OF CAR-T CELLS**
THERAPEUTISCHES HYDROGEL ZUR ABGABE VON CAR-T-ZELLEN
HYDROGEL THÉRAPEUTIQUE POUR L'ADMINISTRATION DE LYMPHOCYTES T CAR

(30) Priority: 23.07.2020 US 202063055738 P
(43) Date of publication of application: 31.05.2023
(73) Proprietor: The Regents of the University of California, Oakland, CA 94607-5200 (US); North Carolina State University, Raleigh, North Carolina 27606 (US)
(72) Inventor: GU, Zhen, Los Angeles, California 90095-7191 (US); HU, Quanyin, Los Angeles, California 90095-7191 (US); LI, Hongjun, Los Angeles, California 90095-7191 (US)
(74) Representative: Ipsilon Luxembourg
(86) International application number: PCT/US2021/042845
(87) International publication number: WO 2022/020644

(56) References cited:
- US-A1- 2008 193 536
- US-A1- 2018 072 811
- US-A1- 2020 181 227
- US-A1- 2020 206 265
- ATIK AHMET F ET AL: "Hyaluronic acid based low viscosity hydrogel as a novel carrier for Convection Enhanced Delivery of CAR T cells", JOURNAL OF CLINICAL NEUROSCIENCE, vol. 56, 21 July 2018 (2018-07-21), pages 163 - 168, XP085502073, ISSN: 0967-5868, DOI: 10.1016/J.JOCN.2018.06.005
- WANG KAI ET AL: "GD2-Redirected T Cells Encapsulated in Hydrogel Control Retinoblastoma and Protect Mouse Vision", MOLECULAR THERAPY, vol. 28, no. 4 Suppl. 1, 339, 28 April 2020 (2020-04-28), 23rd Annual Meeting of the American Society for Gene and Cell Therapy; virtual; 12-15 May 2020, pages 154 - 155, XP093185828, ISSN: 1525-0016, DOI: 10.1016/j.ymthe.2020.04.019
- WANG KAI ET AL: "GD2-specific CAR T cells encapsulated in an injectable hydrogel control retinoblastoma and preserve vision", NATURE CANCER, vol. 1, no. 10, 12 October 2020 (2020-10-12), pages 990 - 997, XP093185820, ISSN: 2662-1347, DOI: 10.1038/s43018-020-00119-y
- HU QUANYIN ET AL: "Conjugation of haematopoietic stem cells and platelets decorated with anti-PD-1 antibodies augments anti-leukaemia efficacy", NATURE BIOMEDICAL ENGINEERING, vol. 2, no. 11, 29 October 2018 (2018-10-29), pages 831 - 840, XP036629635, DOI: 10.1038/S41551-018-0310-2
- RUAN HUITONG ET AL: "Strategies of Combination Drug Delivery for Immune Checkpoint Blockades", ADVANCED HEALTHCARE MATERIALS, vol. 8, no. 4, 1801099, 13 December 2018 (2018-12-13), XP072464340, ISSN: 2192-2640, DOI: 10.1002/ADHM.201801099
- JIANG TIANYUE ET AL: "Gel-Liposome-Mediated Co-Delivery of Anticancer Membrane-Associated Proteins and Small-Molecule Drugs for Enhanced Therapeutic Efficacy", ADVANCED FUNCTIONAL MATERIALS, vol. 24, no. 16, 2 January 2014 (2014-01-02), pages 2295 - 2304, XP072291450, ISSN: 1616-301X, DOI: 10.1002/ADFM.201303222
- CHAO WANG, WUJIN SUN, YANQI YE, QUANYIN HU, HUNTER N. BOMBA, ZHEN GU: "In situ activation of platelets with checkpoint inhibitors for post-surgical cancer immunotherapy", NATURE BIOMEDICAL ENGINEERING, vol. 1, no. 2, 1 February 2017 (2017-02-01), XP055682637, DOI: 10.1038/s41551-016-0011
- HU QUANYIN; LI HONGJUN; ARCHIBONG EDIKAN; CHEN QIAN; RUAN HUITONG; AHN SARAH; DUKHOVLINOVA ELENA; KANG YANG; WEN DI; DOTTI GIANPIE: "Inhibition of post-surgery tumour recurrence via a hydrogel releasing CAR-T cells and anti-PDL1-conjugated platelets", NATURE BIOMEDICAL ENGINEERING, NATURE PUBLISHING GROUP UK, LONDON, vol. 5, no. 9, 26 April 2021 (2021-04-26), London , pages 1038 - 1047, XP037581306, DOI: 10.1038/s41551-021-00712-1

## Description

### Technical Field

The technical field generally relates to hydrogel materials used for the delivery of Chimeric Antigen Receptor (CAR)-engineered T cells (CAR-T cells) to diseased tissue. In particular, the technical field pertains to a hydrogel material that acts as a reservoir for both CAR-T cells and platelets decorated with the anti-programmed death-ligand 1 (PDL1) blocking antibody (aPDL1). The hydrogel may be delivered to the tumor resection cavity or the like.

### Statement Regarding Federally Sponsored Research and Development

This invention was made with government support under Grant Number CA234343, awarded by the National Institutes of Health. The government has certain rights in the invention.

### Background

Despite multiple treatment modalities involving surgery, radiotherapy and chemotherapy, tumor relapse frequently occurs in patients with resectable solid tumors. One reason accounting for tumor recurrence is the post-surgery inflammation that can trigger tumor growth and metastasis. Immunotherapy with immune checkpoint blockades unleashes tumor reactive T cells by blocking the immune checkpoint pathways and may prevent tumor recurrence after surgery. However, systemic administration of checkpoint blockades promote sustained clinical responses in less than 20% of patients with immunogenic tumors. Furthermore, checkpoint blockades are ineffective in tumors characterized by low burden of somatic mutations generating neoantigens and endogenous T cell responses. Finally, side effects including autoimmune diseases secondary to checkpoint blockades remain a concern.

An alternative way to provide tumor specific T cells relies on the adoptive transfer of engineered T cells. Adoptive transfer of T cells expressing a chimeric antigen receptor (CAR) has been demonstrated to be particularly effective in patients with B-cell derived malignancies. In contrast, the application of CAR-T cells in solid tumors remains challenging at least in part because the tumor microenvironment in solid tumors is highly immunosuppressive and induces exhaustion of CAR-T cells.

US 2018/072811 A discloses CAR-T cells targeting chondroitin sulphate proteoglycan 4 (CSPG4) for the treatment of solid tumors.

### Summary

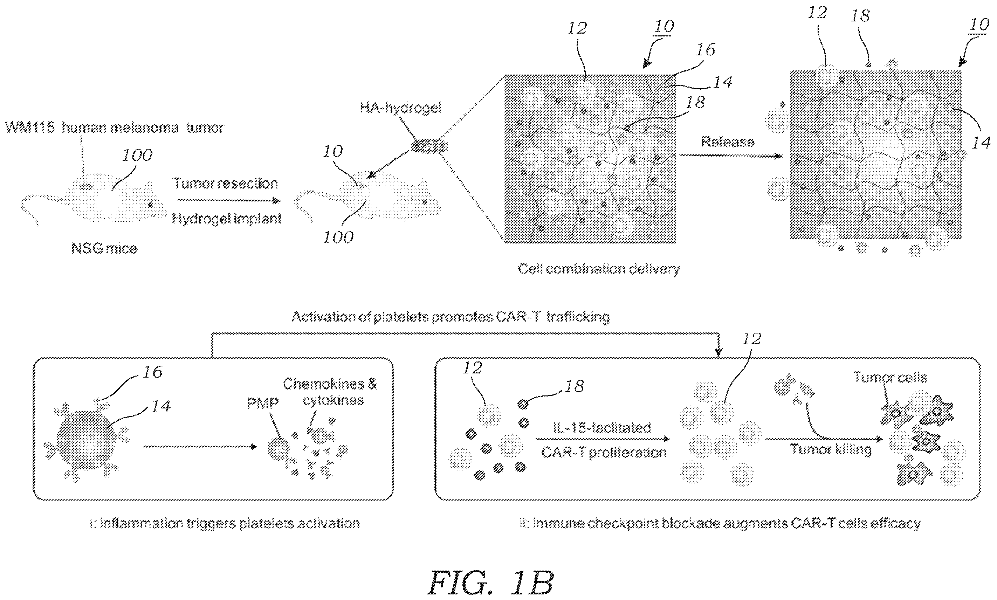

A biocompatible therapeutic hydrogel material is disclosed that functions as a reservoir that encapsulates CAR-T cells targeting the human chondroitin sulphate proteoglycan 4 (CSPG4) (CSPG4.CAR) and can be implanted in or otherwise delivered to the tumor resection recess, cavity, or void in a melanoma tumor model (FIG. 1B). The invention is defined by the attached set of claims. CSPG4 was selected as target antigen because it is highly expressed in human melanoma, while with restricted expression in normal cells. The implantable or deliverable gel acts as a reservoir to concentrate and gradually release CAR-T cells upon tumor surgical resection. To maintain the activity and proliferation ability of CAR-T cells, the cytokine interleukin 15 (IL-15) was also encapsulated in nanoparticles loaded in the hydrogel. Interleukin 7 (IL-7) may also be used. Cytokines facilitate the proliferation of CAR-T cells. Furthermore, human platelets conjugated with the anti-PDL1 blocking antibody (aPDL1) were also contained in the hydrogel to block the PD-1/PDL1 pathway. The inflammation occurring post-surgery triggers the activation of platelets, leading to the formation of platelet-derived microparticles (PMPs) releasing aPDL1 that bind to the tumor cells and block PDL1. Taken together, the developed hydrogel creates a favorable milieu in which CAR-T cells eradicate residual tumor cells post-surgery and prevent tumor recurrence.

The hydrogel material is able to release CAR-T cells in a sustained manner, minimizing the rapid exhaustion of CAR-T cells with bolus delivery. Furthermore, the platelets could be activated by the inflammation condition created at the wound site after tumor removal. The platelet activation could facilitate the release of aPDL1 in a form of platelet-derived microparticles, promoting the blockade of immune checkpoint pathway. This combination of immune checkpoint blockade and CAR-T achieves synergistic treatment efficiency to prevent tumor recurrence.

In one embodiment, a therapeutic hydrogel material is disclosed that is applied to or otherwise delivered to mammalian tissue at the site of a tumor resection. The hydrogel, in one embodiment, is a hyaluronic acid (HA) based hydrogel. The hydrogel is loaded with CAR-T cells and human platelets that are conjugated with anti-PDL1 blocking antibody. In some embodiments, the hydrogel is further loaded with a cytokine (e.g., IL-15) encapsulated in nanoparticles that are also loaded into the hydrogel. In one embodiment, the CAR-T cells target the human chondroitin sulphate proteoglycan 4 (CSPG4) (CSPG4.CAR).

In one embodiment, the HA-based hydrogel is formed with *N,N-*methylenebisacrylamide (MBA) (MBA:HA, 1:5, w:w) and a photo-initiator Irgacure^{™} 2959 (0.1%, w:v) is subject to radical polymerization via UV radiation exposure. The hydrogel may then be lyophilized. To load the CAR-T cells and the platelets (with aPDL1), respective solutions containing the same may be combined with the lyophilized hydrogel (on ice). The cytokine may be similarly loaded into the hydrogel.

To use the therapeutic hydrogel, a solid or other tumor is resected or removed from mammalian tissue. The resection or removal of the tumor forms a recess, cavity, or void in some cases. The hydrogel material containing the CAR-T cells, conjugated platelets, and optional cytokine(s) are then delivered and/or located in the resection recess, cavity, or void. In one embodiment, the hydrogel may be injected into the recess, cavity, or void using an injection device (e.g., syringe, catheter, or the like). The therapeutic hydrogel may in some embodiments, be biodegradable over a period of time.

### Brief Description of the Drawings

FIG. 1A illustrates human skin tissue with a recess, cavity, or void that is filled with the therapeutic hydrogel material. The therapeutic hydrogel material includes CAR-T cells, human platelets conjugated with anti-PDL1 blocking antibody, and optional nanoparticles loaded with one or more cytokines.
FIG. 1B is a schematic illustration of the tumor resection model and implantation of the engineered HA hydrogel. Platelets activated during the wound healing process after surgery release aPDL1 in the format of PMP-aPDL1. PMP, platelet-derived microparticles; MHC, major histocompatibility complex; TCR, T-cell receptor; NP, nanoparticle; HA, hyaluronic acid.
FIG. 1C illustrates confocal images of CAR-T cells and platelet-aPDL1 encapsulated in the hydrogel (CAR-T-P-aPDL1@gel). CAR-T cells and platelets were labeled with Celltracker Green and rhodamine B, respectively. Hoechst 33254 was used to stain nuclei. Cell number: 2×10⁶ CAR-T cells and 1×10⁷ platelets.
FIG. 1D illustrates cyro scanning electron microscope (cyroSEM) images of CAR-T-P-aPDL1@gel. Cell number: 2×10⁶ CAR-T cells and 1×10⁷ platelets. Scale bar: 10 µm.
FIG. 1E illustrates confocal images of the live/dead assay of CAR-T cells released from the hydrogel. Live cells and dead cells were labeled with green fluorescence and red fluorescence, respectively. Scale bar: 100 µm.
FIG. 1F illustrates a histogram of human IFNy released by CAR-T cells encapsulated in the hydrogel. Data are presented as mean ± s.d. (n=3).
FIG. 1G illustrates a histogram of human IL-2 released by CAR-T cells encapsulated in the hydrogel. Data are presented as mean ± s.d. (n=3).
FIG. 1H illustrates *in vivo* degradation of the HA hydrogel labelled with Cy5.5 at week 0 and week 8 (n=3) as measured by fluorescence signals via *in vivo* imaging system (IVIS).
FIG. 2A illustrates a graph showing the release profile of CAR-T cells and platelets from the hydrogel (CAR-T-P-aPDL1@gel) *in vitro.* Cell number: 1×10⁷ CAR-T cells and 1×10⁷ platelets. Data are presented as mean ± s.d. (n = 3).
FIG. 2B illustrates images showing the movement track of platelets and CAR-T cells in the hydrogel as imaged by confocal microscope. Each color represents the movement track of one cell. The movement of CAR-T cells and platelets was monitored every 30 seconds for 30 mins. Cell number: 2×10⁶ CAR-T cells and 1×10⁷ platelets.
FIG. 2C is a histogram showing the migration distance of platelets and CAR-T cells in the hydrogel after 30 minutes. Data are presented as mean ± s.d. (n = 400).
FIGS. 2D-2F illustrates flow cytometry plots (FIG. 2D) and quantification of tumor cells (FIG. 2E) and T cells (FIG. 2F) in tumor cell and T cell coculture for 72 hours. T@gel, control T cells in the hydrogel; T-Platelet@gel, control T cells and platelets in the hydrogel; CAR-T@gel, CAR-T cells in the hydrogel; CAR-T-P-aPDL1@gel, CAR-T cells and aPDL1-loaded platelets in the hydrogel. Data are presented as mean±s.d. (n=3). Cell number: 3.3×10⁵ CAR-T cells, 1×10⁷ platelets and 1×10⁶ WM115 cells. The amount of aPDL1 is 1 µg.
FIGS. 2G and 2H show histograms of human IFNy (FIG. 2G) and IL-2 (FIG. 2H) released by CAR-T cells during the co-cultures illustrated in FIG. 2D. Data are presented as mean ± s.d. (n = 3) **P*<0.05, one-way ANOVA, followed by Tukey's HSD post hoc test.

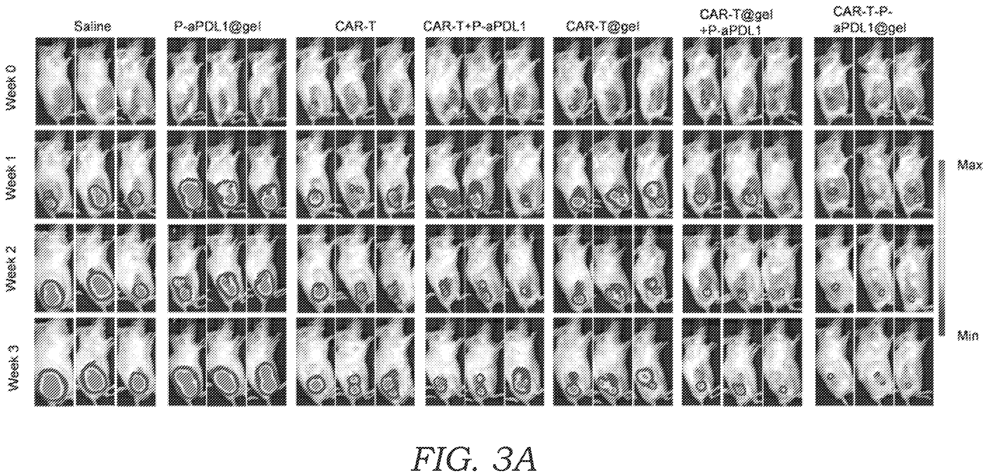

FIG. 3A illustrate representative tumor bioluminescence images of treated mice under different conditions. Saline, saline solution; aPDL1@gel, aPDL1 encapsulated in the hydrogel; CAR-T, CAR-T cells directly inoculated into the resection cavity; CAR-T+P-aPDL1, CAR-T cells and aPDL1 directly inoculated into the resection cavity; CAR-T@gel, CAR-T cells encapsulated in the hydrogel; CAR-T@gel + aPDL1, CAR-T cells and aPDL1 encapsulated in the hydrogel; CAR-T-P-aPDL1@gel, CAR-T cells, platelets and aPDL1 encapsulated in the hydrogel. In all experimental groups IL-15-NP (IL-15: 1 µg) were included. Cell number: 2×10⁶ CAR-T cells and 1×10⁷ platelets. The amount of aPDL1 and IL-15 were both 1 µg.
FIG. 3B shows region-of-interest analysis of tumor bioluminescence intensities from the images of FIG. 3A.
FIG. 3C is a histogram showing the comparison of tumor bioluminescence intensities at week 3 after treatment. Data are presented as mean ± s.d. (n = 6 mice/group) **P<0.01, one-way ANOVA, followed by Tukey's HSD post hoc test.
FIG. 3D illustrates a histogram showing the summary of the tumor volume at week 3 after treatment. Data are presented as mean ± s.d. (n = 6 mice/group) **P<*0.05, *** *P<*0.001, one-way ANOVA, followed by Tukey's HSD post hoc test.
FIG. 3E shows images of representative tumors after 3 weeks. 1. saline, 2. P-aPDL1@gel, 3. CAR-T, 4. CAR-T+P-aPDL1, 5. CAR-T@gel, 6. CAR-T@gel +P-aPDL1, and 7. CAR-T-P-aPDL1. Scale bar: 1 cm.

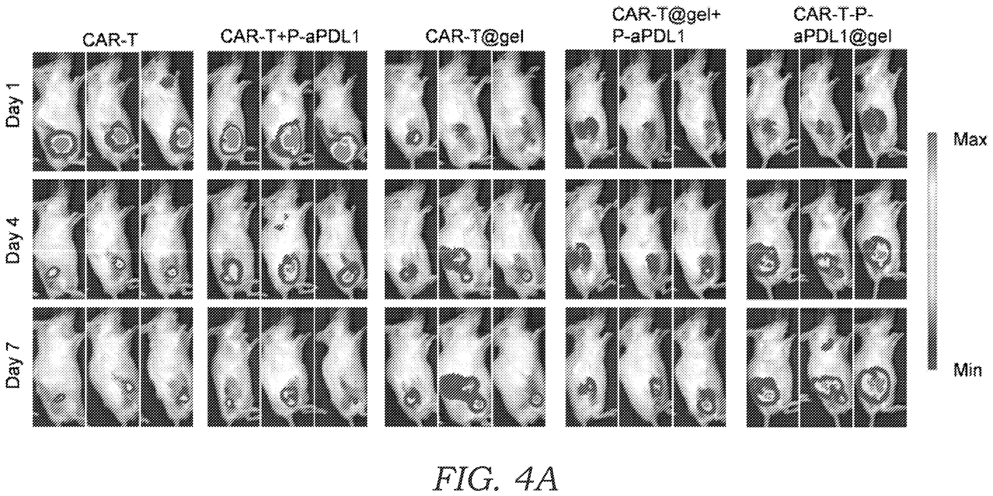

FIG. 4A illustrate representative T cell bioluminescence images of treated mice under different conditions. CAR-T, CAR-T cells directly inoculated into the resection cavity; CAR-T + aPDL1, CAR-T cells and aPDL1 directly inoculated into the resection cavity; CAR-T@gel, CAR-T cells encapsulated in the hydrogel; CAR-T@gel + aPDL1, CAR-T cells and aPDL1 encapsulated in the hydrogel; CAR-T-P-aPDL1@gel, CAR-T cells, platelets and aPDL1 encapsulated in the hydrogel. In all experimental groups IL-15-NP (IL-15: 1 µg) were included. Cell number: 2×10⁶ CAR-T cells and 1×10⁷ platelets. The amounts of aPDL1 and IL-15 were both 1 µg.
FIG. 4B shows region-of-interest analysis of T cell bioluminescence of the images of FIG. 4A. Data are presented as mean ± s.d. (n = 6 mice/group) ** P<0.01, one-way ANOVA, followed by Tukey's HSD post hoc test.
FIG. 4C illustrates a confocal image of CAR-T-P-aPDL1@gel 72 hours after treatment. CAR-T cells and platelets were labeled with Celltracker Orange and rhodamine B, respectively. Cell number: 2×10⁶ CAR-T cells and 1×10⁷ platelets. The amounts of aPDL1 and IL-15 were both 1 µg. Scale bar on the left: 100 µm. Scale bar on the right: 20 µm.
FIGS. 4D and 4E illustrate histograms of human IFNy (FIG. 4D) and IL-2 (FIG. 4E) detected *in vivo* within the tumor at week 1 after treatment. Data are presented as mean ± s.d. (n = 6) **P*<0.05, ***P*<0.01, ****P<*0.001, one-way ANOVA, followed by Tukey's HSD post hoc test.
FIG. 5A illustrates representative tumor bioluminescence images of treated mice under different conditions. Saline, saline solution; CAR-T + aPDL1, CAR-T cells and aPDL1 directly inoculated into the resection cavity; CAR-T-P-aPDL1@gel, CAR-T cells, platelets and aPDL1 encapsulated in the hydrogel. In all experimental groups IL-15-NP (IL-15: 1 µg) were included. Cell number: 2×10⁶ CAR-T cells and 1×10⁷ platelets. The amounts of aPDL1 and IL-15 were both 1 µg.
FIG. 5B is a graph showing tumor growth measured on the left side after treatment of the primary tumor. Data are presented as mean ± s.d. (n = 6) ***P*<0.01, two-way ANOVA.
FIG. 5C are images showing representative tumors at day 18. Scale bar: 1 cm.
FIG. 6 shows fluorescence images showing aPDL1 conjugated to platelets. aPDL1 was stained with FITC and platelets were stained with rhodamine B. Scale bar is 50 µm.
FIG. 7 shows representative flow cytometry histograms of platelets alone (left) and platelets conjugated with aPDL1 (P-aPDL1) (right) upon staining with the FITC-labeled anti-IgG antibody.
FIG. 8 shows images of collagen binding capability of naive platelets and P-aPDL1. Platelets were labeled with WGA-Alex Fluor 594 for imaging. Scale bar: 50 µm.
FIG. 9 illustrates a graph of Cumulative aPDL1 release *in vitro* from P-aPDL1 in the native status or after activation. Platelets were activated with 0.5 U ml⁻¹ thrombin. The data are presented as means ± s.d. (n=3).
FIG. 10 illustrates binding of PMP-aPDL1 to WM115 tumor cells in a trans-well assay. Platelets were placed in the upper chamber with the pore size of 1 µm and WM115 tumor cells were seeded in the bottom chamber. Platelets were stained with WGA-Alex Fluor 594 and WM115 tumor cells were labeled with WGA-Alex Fluor 488 and Hoechst. Scale bar: 20 µm.
FIG. 11 shows an image illustrating the morphologies of the lyophilized and cell-loaded HA hydrogel.
FIG. 12 illustrates flow cytometry analysis showing CAR and GFP expression in CAR-T cells specific for the CSPG4 antigen co-transduced with a vector encoding the fusing protein GFP-Firefly luciferase.
FIGS. 13A-13C illustrate the characterization of the IL-15-NP *in vitro.* (FIG. 13A) The hydrodynamic size of IL-15-NP was measured by Dynamic Light Scattering (DLS). (FIG. 13B) The morphology of IL-15-NP was imaged by transmission electron microscope (TEM). (FIG. 13C) Release profile of IL-15-NP *in vitro* (n=3). Data are presented as mean±s.d.
FIG. 14 illustrates the loading efficiency of CAR-T cells and IL-15-NP in the hydrogel. CAR-T cells and IL-15-NP with Cy5.5 fluorophore were loaded in the hydrogels in a 48-well plate. After 15 min, hydrogels containing CAR-T cells and IL-15-NP were transferred to another well. Fluorescence intensity in the original wells was measured to quantify cells/NP leaked out from the hydrogel. Data are presented as mean ± s.d. (n=5)
FIG. 15 illustrates degradation of the Cy5.5-labeled HA-hydrogel *in vivo* measured by fluorescence signals via *in vivo* bioluminescence (IVIS). The hydrogel was implanted in the cavity after tumor resection and the wound was closed using an Autoclip wound closing system.
FIG. 16 illustrates CAR-T cell release profile *in vivo* after gel implantation within the tumor resection cavity. CAR-T cells were labeled with CellTracker^{™} Deep Red for the *in vivo* release quantification. Each day after implantation, the hydrogel was removed and the CAR-T cells released and accumulated within the tumor were quantified. Data are presented as mean ± s.d. (n = 6 mice/group).
FIG. 17 illustrates proliferation of CFSE-labeled CAR-T cells. CFSE dilution was measured by flow cytometry. CAR-T cells were stimulated with CSPG4⁺ tumor cells. Non-transduced T cells and T cell specific for the CD19 antigen (CD19-CAR-T) were used as negative controls.
FIG. 18 illustrates PDL1 expression in WM115 tumor cells treated *in vitro* with either CAR-T cells or CAR-T cells + platelets. Expression of PDL1 was assessed by flow cytometry. WM115 indicates the expression of PDL1 in WM115 tumor cell alone, while Isotype control indicates the histogram of WM115 tumor cells stained with the isotype control antibody.
FIGS. 19A-19B illustrates confocal images showing the expression of CSPG4 (FIG. 19A) and PDL1 (FIG. 19B) in WM115 tumor cells growing *in vivo.* Tumors were resected 2 weeks after tumor cell inoculation and mechanically disrupted for flow cytometry analysis. Scale bar: 100 µm.
FIG. 20 illustrates flow cytometry histograms showing the expression of PDL1 in tumors removed from control mice and mice treated with CAR-T@gel. Tumors were removed 2 weeks after treatment with CAR-T@gel. An irrelevant antibody was used as isotype control.
FIG. 21A illustrates representative tumor bioluminescence in mice treated with T-Platelet@gel, CAR-T-P-isotype antibody@gel, CAR-T@gel + Systemic aPDL1 and CAR-T cells loaded in the hydrogel and aPDL1. For the systemic administration of aPDL1, 10 µgs were injected i.v. for three consecutive days.
FIG. 21B shows a graph comparison of the tumor bioluminescence intensities at week 3 after treatment. Data are presented as mean ± s.d. (n = 6 mice/group) **P* <0.05, **P<0.01, one-way ANOVA, followed by Tukey's HSD post hoc test.
FIG. 21C shows representative images of macroscopic tumors collected 3 weeks after treatment. 1. T-Platelet@gel, 2. CAR-T-P-isotype antibody@gel, 3. CAR-T@gel + Systemic aPDL1, 4. CAR-T-P-aPDL1@gel. Scale bar: 1 cm.
FIG. 22 shows representative bioluminescence of CAR-T cells in vivo. Tumor bearing mice underwent tumor resection and local treatment with CAR-T cells or CAR-T cells + aPDL1 directly inoculated into the resection cavity or encapsulated CAR-T cells (CAR-T@gel, CAR-T@gel + aPDL1 or CAR-T-P-aPDL1@gel). For this experiment CAR-T cells were also transduced with the vector encoding GFP-Firefly luciferase, and T cell bioluminescence was detected weekly by IVIS.
FIG. 23A illustrates representative tumor bioluminescence images of CAR-T cells and CAR-T@gel in the mouse model designed to demonstrate the abscopal effects. WM115 tumor cells (5×10⁶ cells) were inoculated in both right and left flanks. When tumors reached 100 mm³, tumors were removed on the left side and the hydrogel was implanted, while tumors on the right side were left untouched. Tumor growth was monitored by in vivo bioluminescence IVIS.
FIG. 23B shows a graph of the summary of the tumor growth measured on the right side after treatment of the primary tumor. Data are presented as mean ± s.d. (n = 6) ***P<0.001, two-way ANOVA.
FIG. 23C illustrates images of the tumors removed from the right side at day 18. Scale bar: 1 cm.
FIG. 24 illustrates fold changes of chemokines before and after tumor resection. Mice were engrafted with WM115 tumor cells. Tumors were removed 2 weeks after tumor cell inoculation. Twenty-four hours after surgery, tissues were harvested around the cavity. Tissues were mechanically disrupted to cell suspension to measure chemokine. Data are presented as mean ± s.d. (n = 6 mice/group).
FIG. 25A illustrates representative flow cytometry plots of CAR-T cells detected in the peripheral blood of WM115-bearing mice after treatment with saline, CAR-T+P-aPDL1 or CAR-T-P-aPDL1@gel.
FIG. 25B illustrates the quantitative analysis of CAR-T cells in the peripheral blood. Data are presented as mean ± s.d. (n = 5 mice/group) ***P<0.001, one-way ANOVA, followed by Tukey's HSD post hoc test.
FIG. 25C illustrates representative flow cytometry plots of CAR-T cells detected in the distant tumor of WM115-bearing mice after treatment with saline, CAR-T+P-aPDL1 or CAR-T-P-aPDL1@gel.
FIG. 25D shows quantitative analysis of CAR-T cells detected in the distant tumor. Data are presented as mean ± s.d. (n = 5 mice/group) ***P<0.001, one-way ANOVA, followed by Tukey's HSD post hoc test.
FIG. 25E illustrates representative confocal images of CD4 and CD8 T cells detected in the distant tumor. Scale bar: 100 µm.
FIG. 26 illustrates a graph showing the measurement of the concentration of hyaluronidase in the normal tissues and tumor. Data are presented as mean ± s.d. (n = 6 mice/group) ****P*=0.0006, unpaired two-tailed Student's *t*-test.
FIG. 27 shows a graph of region-of- interest analysis of tumor bioluminescence immediately after resection. Data are presented as mean ± s.d. (n = 6 mice/group).
FIG. 28 illustrates a graph of the measurement of IL-15 within the tumor at different time points along the 4-week time-course treatment. The dashed line represents the concentration of IL-15 in the non-treated tumor site. Data are presented as mean ± s.d. (n = 6 mice/group).

### Detailed Description of the Illustrated Embodiments

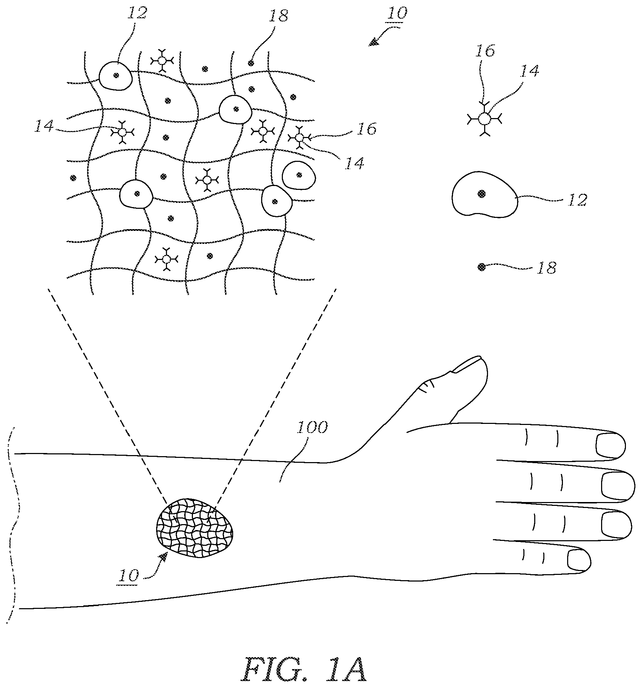

In one embodiment and with reference to FIG. 1A, a therapeutic hydrogel material 10 is disclosed that is applied to a mammalian tissue 100 (seen in FIG. 1A and also FIG. 1B in mouse model) at the site of a tumor or cancerous tissue removal or resection. The therapeutic hydrogel material 10, in one embodiment, is a hyaluronic acid (HA) based hydrogel although other hydrogel materials may be used. These include by way of illustration and not limitation: polysaccharide-based hydrogels (e.g., chitosan); hydrogels formed with acrylic polymers like acrylic acid, acrylamide, hydroxyethyl acrylate; and protein-based hydrogels like fibrinogen and albumin. The therapeutic hydrogel material 10 that is used is biocompatible with mammalian tissue 100 and, in some embodiments, is biodegradable (like the HA-based hydrogel disclosed herein). The therapeutic hydrogel material 10 is loaded with CAR-T cells 12 and human platelets 14 that are conjugated with anti-PDL1 blocking antibody 16. In some embodiments, the therapeutic hydrogel material 10 is further loaded with a cytokine (e.g., IL-15 or IL-7) encapsulated in nanoparticles 18 that are also loaded into the therapeutic hydrogel material 10. The CAR-T cells 12, platelets 14, and cytokines contained within nanoparticles 18 may be added serially to the therapeutic hydrogel material 10 or combined together and loaded into the therapeutic hydrogel material 10. In one embodiment, the CAR-T cells 12 target the human chondroitin sulphate proteoglycan 4 (CSPG4) (CSPG4.CAR). The CAR-T cells 12 may also be modified with other targets (e.g., antigens or biomolecules).

In one embodiment, the HA-based therapeutic hydrogel material 10 is made with N,N-methylenebisacrylamide (MBA) (MBA:HA, 1:5, w:w) and a photo-initiator Irgacure^{™} 2959 (0.1%, w:v) is subject to radical polymerization via UV radiation exposure. The hydrogel material may then be lyophilized. To load the CAR-T cells 12 and the platelets 14 (with aPDL1), respective solutions containing the same may be combined with the lyophilized hydrogel (on ice). The cytokine(s) encapsulated in the nanoparticles 18 may be similarly loaded into the hydrogel material. In some embodiments, the therapeutic hydrogel material 10 contains the CAR-T cells 12 and the platelets 14 conjugated with aPDL1. In other embodiments, the therapeutic hydrogel material 10 contains CAR-T cells 12, the platelets 14 conjugated with aPDL1, and cytokine(s) encapsulated in nanoparticles 18. The cytokines increase the viability and activity of CAR-T cells 12.

To use the therapeutic hydrogel material 10, a solid or other tumor (or cancerous tissue) is first resected or removed from mammalian tissue 100. This may be done using conventional surgical techniques known to those skilled in the art. This may include the use of conventional tools such as scalpels, forceps, scissors, retractors, and the like. Tumor resection may also be performed using a laser or other high energy tool to aid in tumor removal. The resection of the tumor forms a recess, cavity, or void. The therapeutic hydrogel material 10 containing the CAR-T cells 12, conjugated platelets 14, and optional cytokine(s) in the nanoparticles 18 are then located in the resection recess, cavity, or void (FIG. 1A). In one embodiment, the therapeutic hydrogel material 10 may be shaped to roughly accommodate the dimensions of the recess, cavity or void. The therapeutic hydrogel material 10 may in some embodiments, be biodegradable over a period of time. The therapeutic hydrogel material 10 may be supplied as a lyophilized "dry" three-dimensional hydrogel that can be loaded with the CAR-T cells 12, platelets 14, and cytokine(s) in nanoparticles 18 at, for example, the medical facility (e.g., hospital, clinic, or the like) or site of application. Thus, the final product may be made on-site and delivered to the patient. Alternatively, the therapeutic hydrogel material 10 may be provided in a final state (i.e., loaded with CAR-T cells 12, conjugated platelets 14, and optional cytokine(s) inside nanoparticles 18) which can then be used on the mammalian tissue 100.

The therapeutic hydrogel material 10 may be made in a number of sizes and shapes. In this regard, the surgeon or other medical professional can select the appropriate hydrogel to be placed in the recess, cavity, or void. The therapeutic hydrogel material 10 may be provided as part of kit which may include the lyophilized hydrogel material, CAR-T cells 12, conjugated platelets 14, and optional cytokine(s) loaded in nanoparticles 18. Alternatively, the therapeutic hydrogel material 10 may be provided in a ready-to-use format where no mixing or further preparation is needed. The therapeutic hydrogel material 10 may be delivered to the recess, cavity, or void manually or with the aid of a delivery device such as a syringe-type dispenser. The therapeutic hydrogel material 10 may be worked or manipulated to conform or fill the recess, cavity, or void. Optionally, a bandage or wrap may be placed over the filled recess, cavity, or void after delivery of the therapeutic hydrogel material 10.

### Experimental

### Engineering the hydrogel for cell delivery

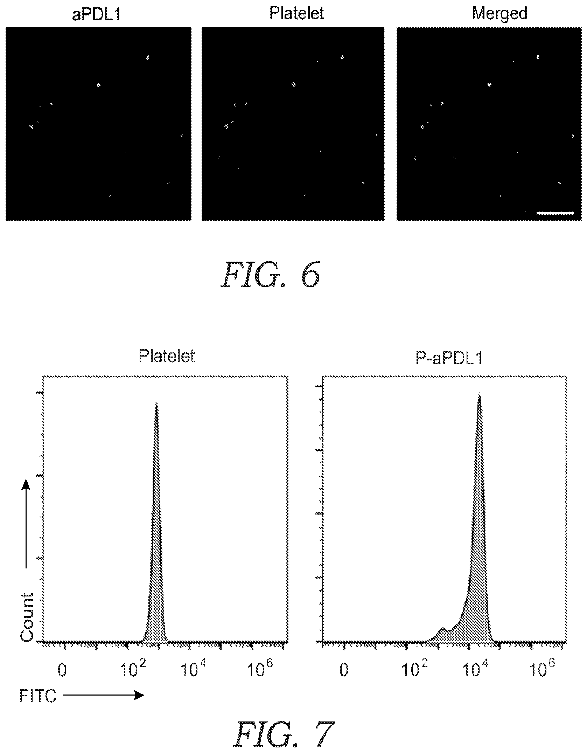

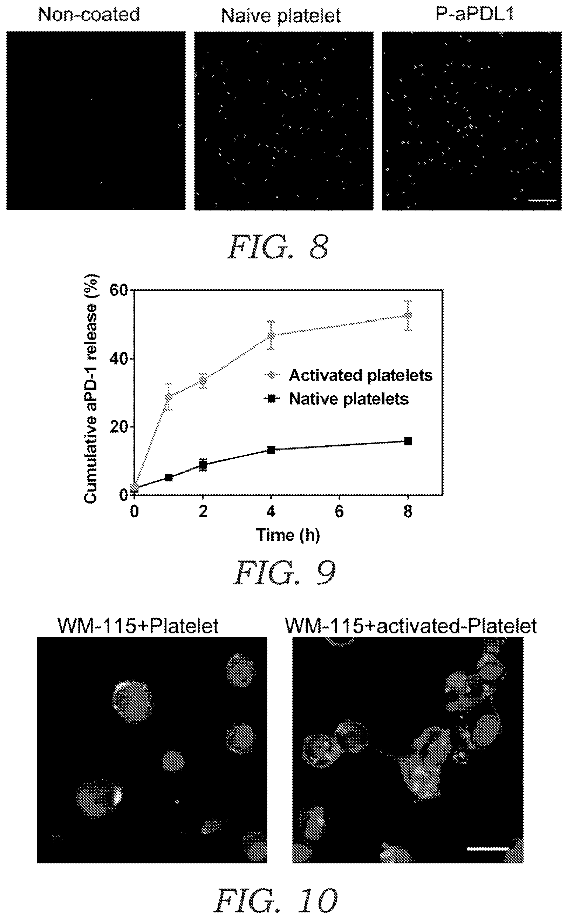

The anti-PDL1 blocking antibody (aPDL1) was covalently conjugated on the cell surface of human platelets (designated as P-aPDL1 14). The binding of aPDL1 to platelets was visualized by colocalization of the fluorescein-labeled aPDL1 antibody and rhodamine B-labeled platelets (FIG. 6) and confirmed by flow cytometry (FIG. 7). Conjugation of aPDL1 did not affect platelet functionality, as evidenced by the conserved collagen binding activity of aPDL1-conjuated platelets (FIG. 8). The release of aPDL1 was accelerated upon platelet activation by thrombin and formation of PMPs (FIG. 9). The interaction between activated platelets and melanoma cells was investigated using a trans-well assay (1 µm micropores) that allows free transportation of PMPs, but not intact platelets. After incubation with thrombin, wheat germ agglutinin (WGA)-Alexa Fluor 594-labeled platelets in the upper chamber were activated and generated PMPs that migrated to the lower chamber in which WGA-Alexa Fluor 488-labeled WM115 melanoma cells were seeded. Co-localization of red florescence and green florescence demonstrated the binding of PMP-aPDL1 and melanoma cells and relocation of aPDL1 on the surface of melanoma cells (FIG. 10). In contrast, negligible binding of aPDL1 to the tumor cells was observed in the control platelets.

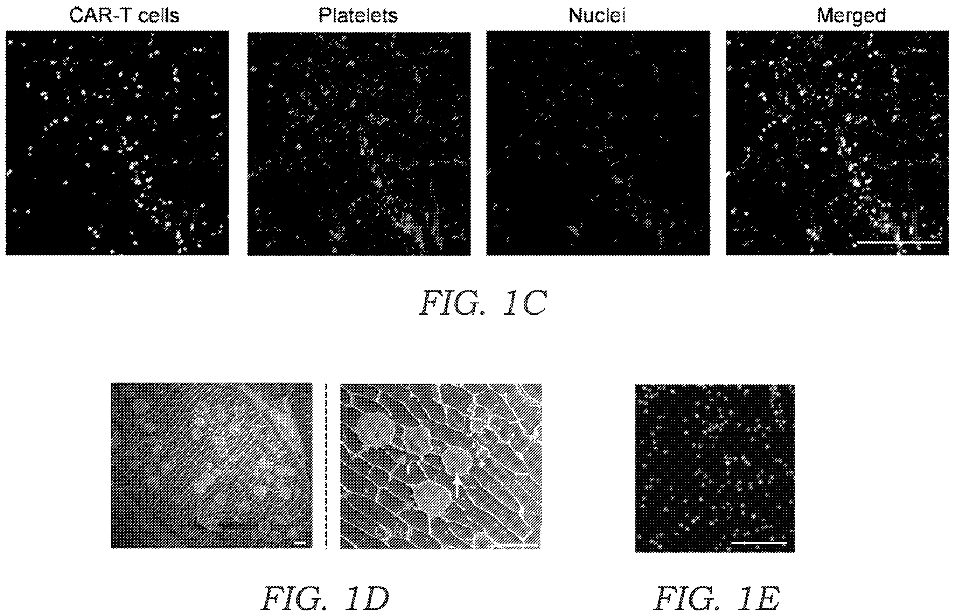

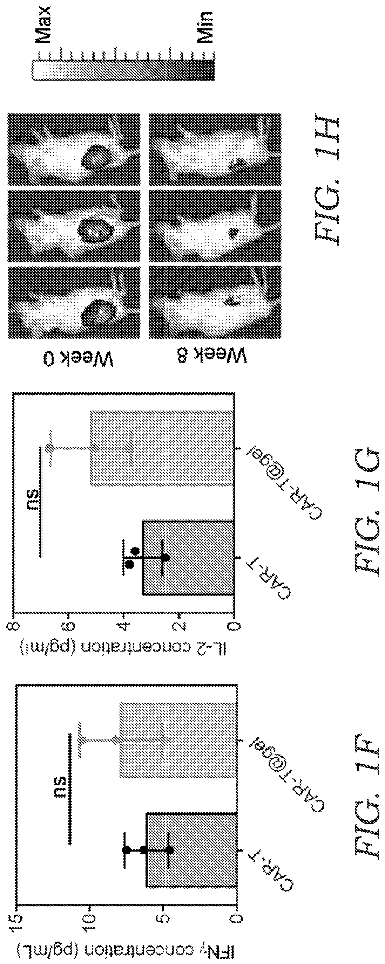

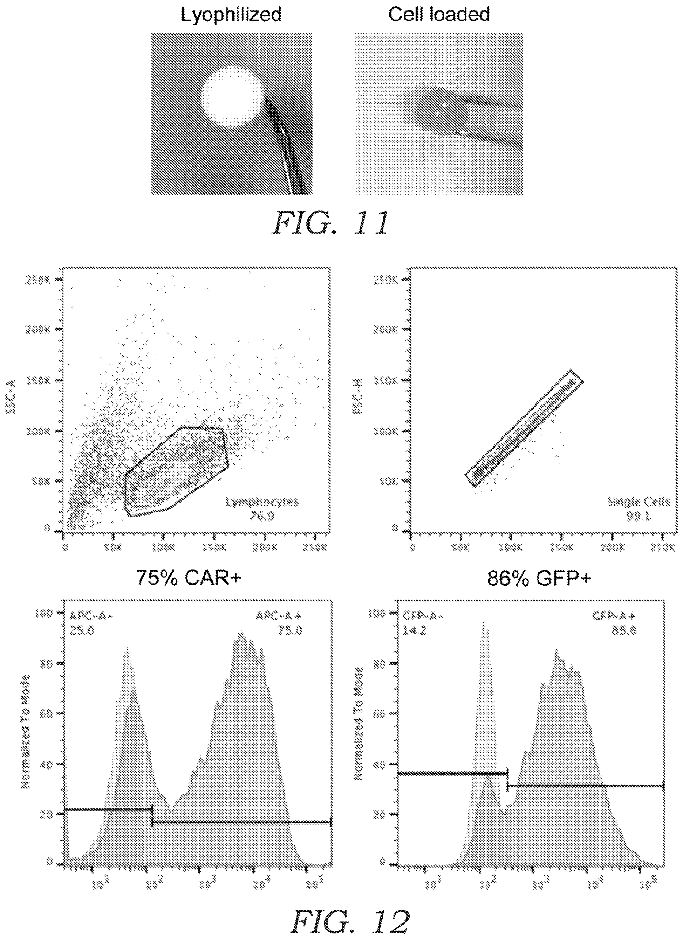

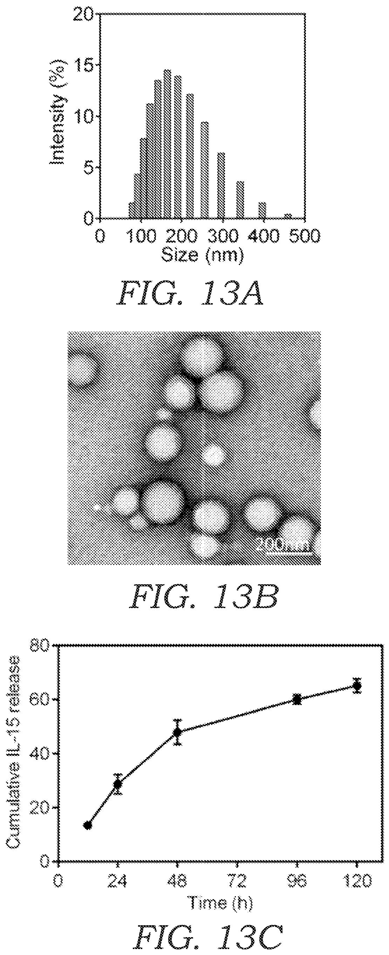

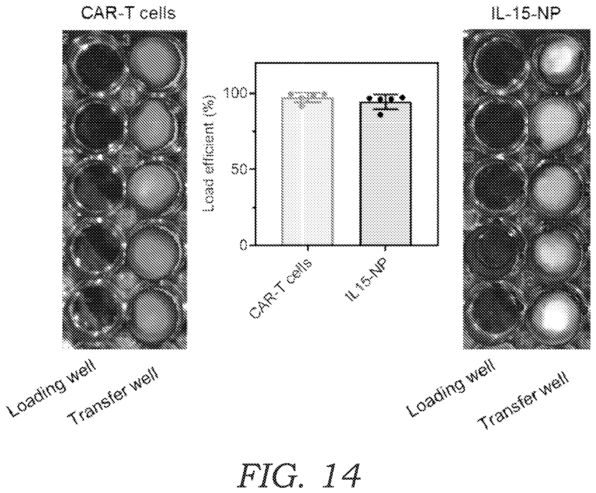

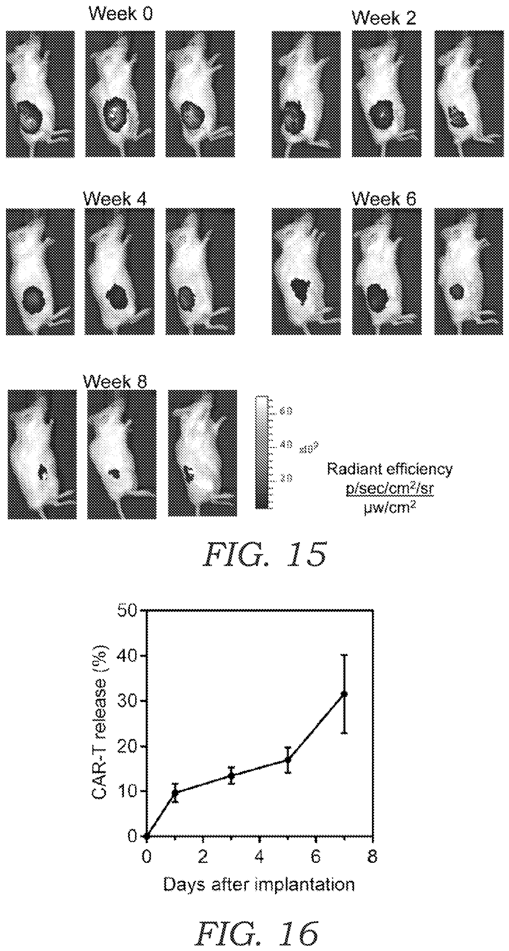

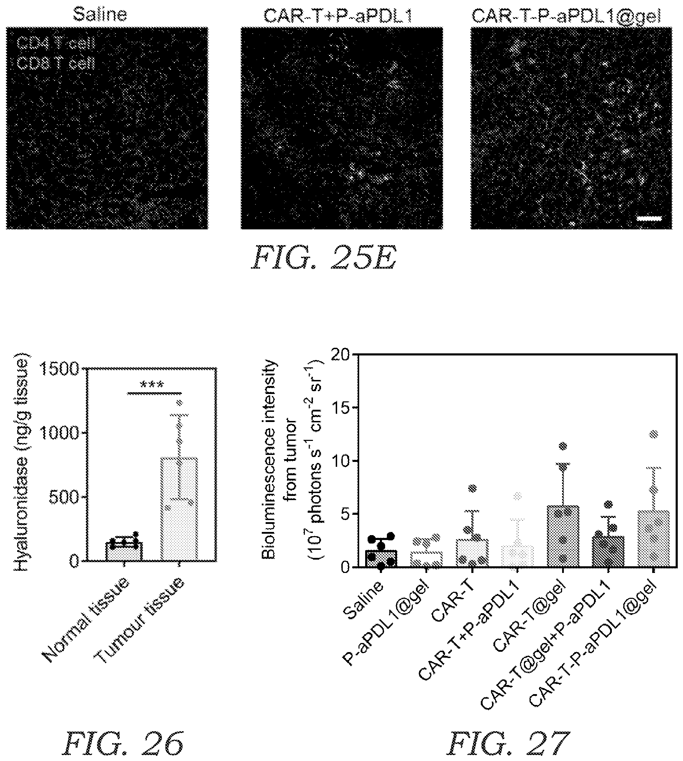

The hydrogel material was generated using the acrylate groups-modified hyaluronic acid (HA) that can be cross-linked by UV irradiation with cross-linker and photo initiator. The HA hydrogel was lyophilized for further storage and use (FIG. 11). CAR-T cells targeting the CSPG4 antigen and the P-aPDL1 were loaded into the hydrogel material (FIG. 12). To support the viability and proliferation of CAR-T cells 12 in the hydrogel matrix material, the cytokine IL-15 was also encapsulated using PLGA nanoparticles 18 (IL15-NPs) that were prepared in water-in-oil-in-water (w/o/w) emulsion. IL-15-NPs 18 displayed a size around 180 nm and a spherical morphology as assessed by transmission electronic microscope (TEM) imaging (FIGS. 13A, 13B), and released IL-15 in a sustained manner with over 60% release at 120 hours (FIG. 13C). No significant leakage of either CAR-T cells 12 or IL-15-NPs 18 loaded into hydrogel was found, as evidenced by 97.3% and 94.6% loading efficacy of CAR-T cells 12 and IL-15-NP 18, respectively (FIG. 14). The distribution of P-aPDL1 14 and CAR-T cells 12 in the hydrogel was demonstrated by confocal microscopy and cyro scan electron microscopy (cyroSEM) (FIGS. 1C, 1D). The hydrogel formulation did not cause toxic effects on CAR-T cells 12 (FIG. 1E). In addition, the HA hydrogel did not induce non-specific activation of CAR-T cells 12, as evidenced by negligible production of IFNγ and IL-2 in the absence of antigen stimulation (FIGS. 1F, 1G). Taken together, these results demonstrate that the HA-hydrogel acts as a reservoir for both CAR-T cells 12 and conjugated platelets 14 carrying the aPDL1 and can integrate molecules delivered by nanoparticles 18. The degradation *in vivo* of the HA-hydrogel was further studied using the In Vivo Imaging System (IVIS). As shown in FIG. 1H and FIG. 15, the HA-hydrogel matrix was gradually degraded by hyaluronidase (FIG. 26) after implantation and minimal signal was detected after 8 weeks demonstrating the optimal biocompatibility and degradability of the HA-hydrogel.

### In vitro CAR-T cell antitumor effects

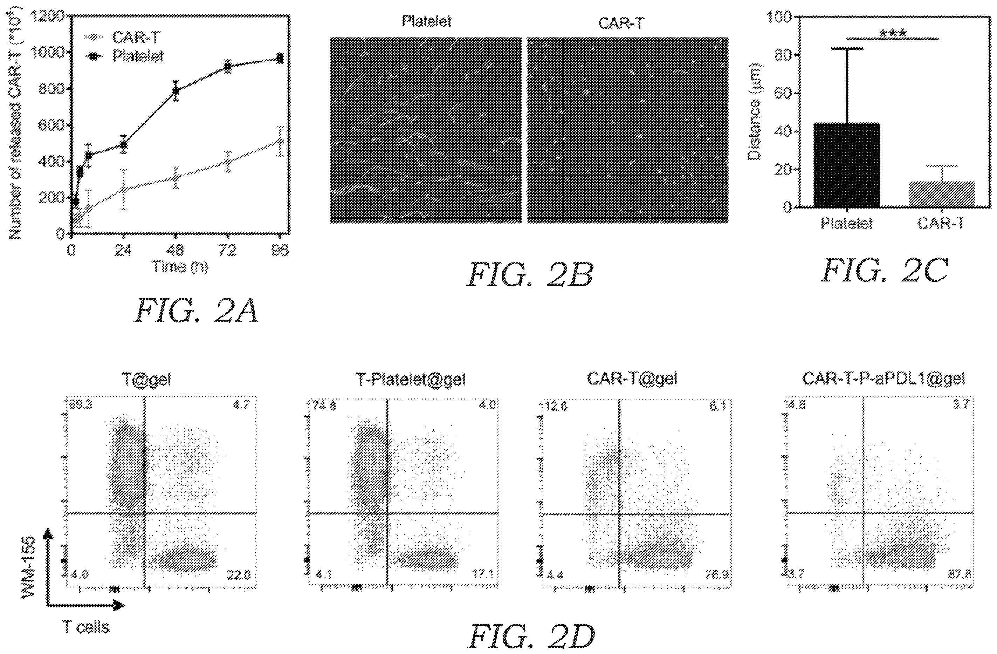

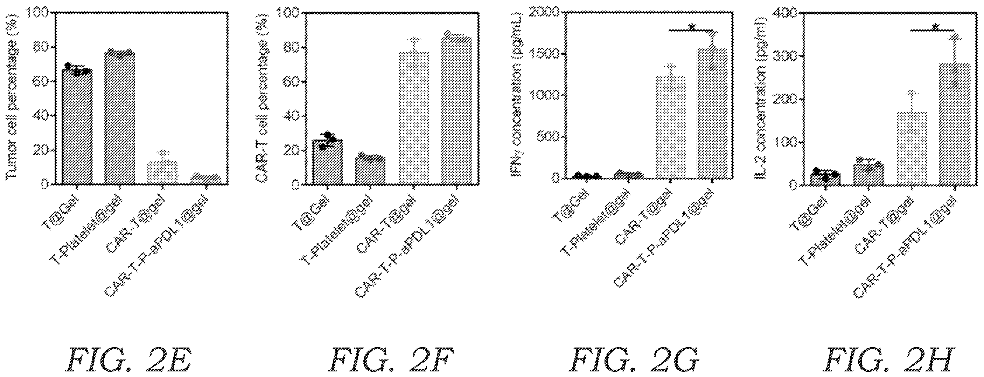

The release profile of both CAR-T cells 12 and the conjugated platelets 14 encapsulated within the hydrogel were investigated. As plotted in the FIG. 2A, both CAR-T cells 12 and platelets 14 were released in a sustained manner. However, platelets 14 showed faster release because at 96 hours, nearly all platelets 14 were released, as compared to 50% release of CAR-T cells 12. The faster release of platelets 14 is likely due to their pronounced motility within the therapeutic hydrogel material 10 as demonstrated by confocal microscopy (FIG. 2B). The average quantitative migration distance of platelets 14 was 4-fold longer than that of CAR-T cells 12 (FIG. 2C). The hydrogel 10 also supported the gradual release of CAR-T cells 12 *in vivo* because 30% of CAR-T cells 12 were released within a week (FIG. 16).

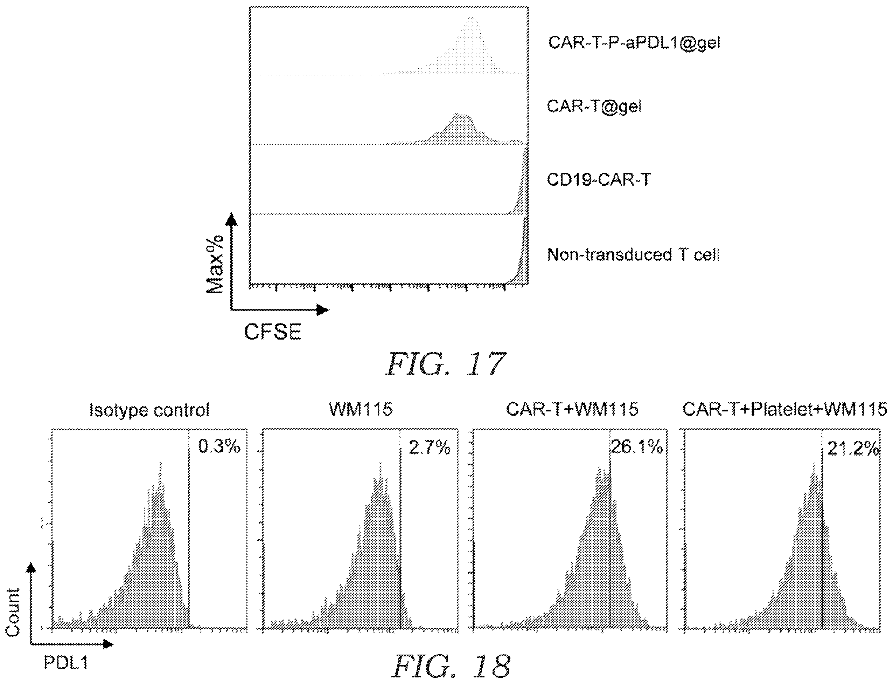

The antitumor activity of CAR-T cells 12 was investigated in a co-culture assay. The hydrogel loaded with P-aPDL1 14 and CAR-T cells 12 was placed in the upper cell strainer (40 µm pore size) and GFP-labeled WM115 tumor cells were seeded in the bottom chamber. After 72 hours, the percentage of WM115 and T cells was not significantly changed in the hydrogels loaded with either control T cells or control T cells and platelets (FIG. 2D). In contrast, the percentage of WM115 cells was decreased (12.7±5.8%) and the percentage of T cells increased (76.8±7.7%) when CAR-T cells 12 were plated in the hydrogel 10 (FIGS. 2D-F). The percentage of WM115 cells further decreased when CAR-T cells 12 were combined with P-aPDL1 (FIGS. 2D-F). CAR-T 12 cells loaded in the hydrogel 10 released IFNy and IL-2 and the cytokine levels were further increased in the presence of P-aPDL1 14 (FIGS. 2G, 2H). CAR-T cells 12 also proliferated in response to tumor cells (FIG. 17). As shown in FIG. 18, WM115 cells exposed to CAR-T cells 12 or CAR-T cells 12 and P-aPDL1 14 showed significant increase on PDL1 expression as compared to other control groups. Collectively, these data demonstrate that the combination of CAR-T cells 12 and P-aPDL1 14 exhibit higher T-cell activation and cytokine release *in vitro.*

### In vivo antitumor activity of the CAR-T-P-aPDL1@gel

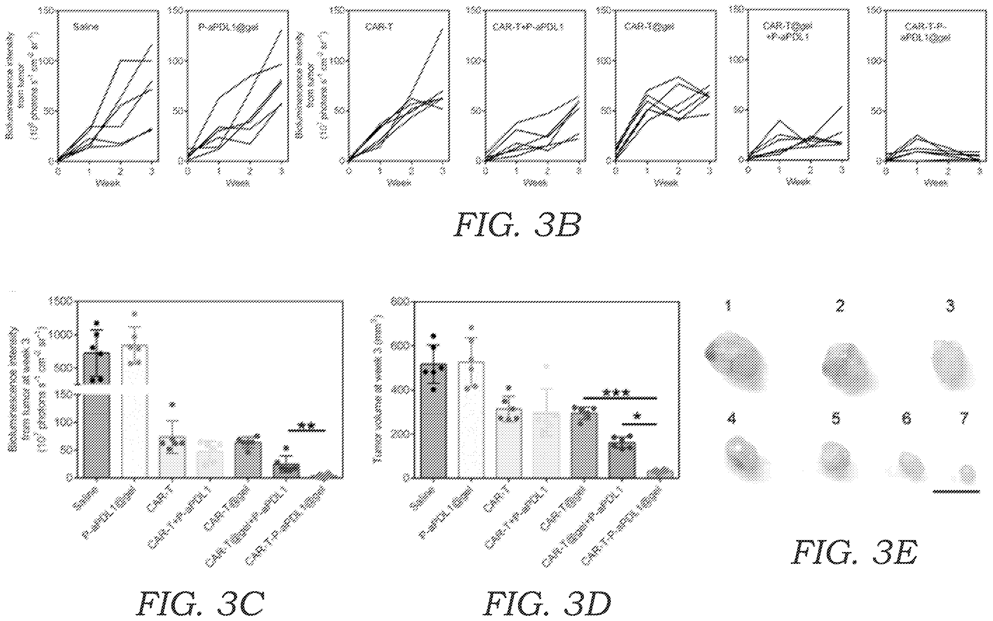

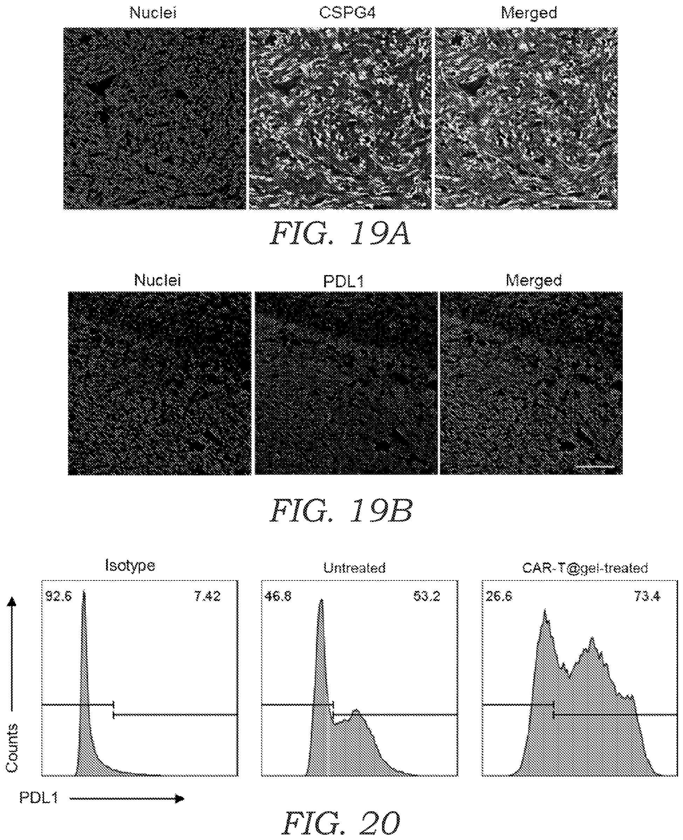

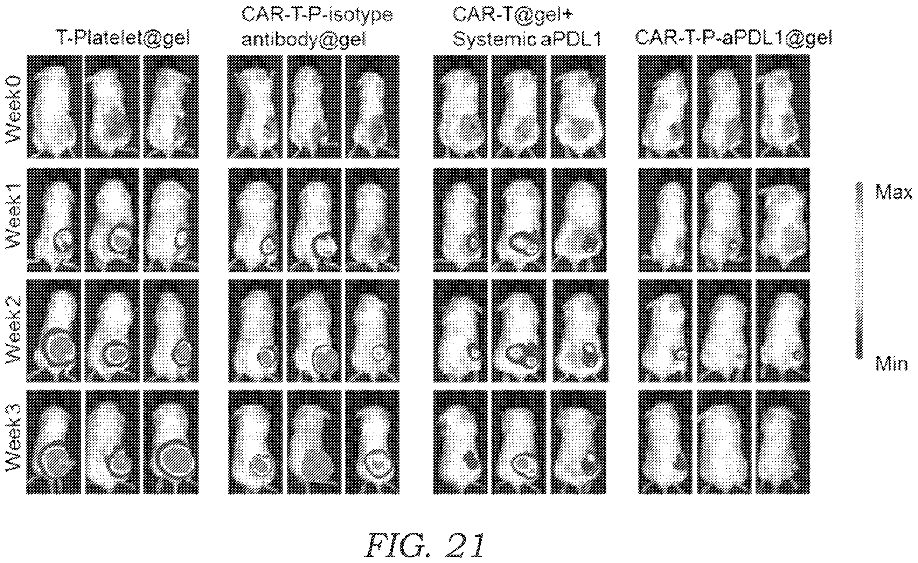

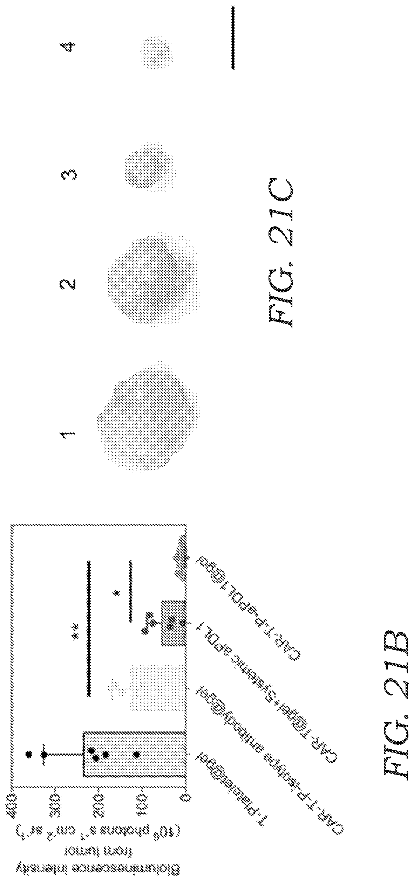

To validate the antitumor effects *in vivo* of the engineered hydrogel 10, NOD-scid IL2Rynull (NSG) mice were inoculated subcutaneously with the luciferase-labeled WM115 tumor cells. Upon engraftment, WM115 tumor cells retain the expression of the target antigen CSPG4 and display PDL1 expression as detected by confocal microscopy (FIGS. 19A-19B). PDL1 expression by tumor cells *in vivo* was also confirmed by flow cytometry (FIG. 20). When the size of the tumors reached to ~150 mm³, tumor masses were partially removed and the sizes of the remaining tumors did not show significant difference based on bioluminescence intensity (FIG. 27). Post tumor removal, mice were treated with saline, non-transduced T cells and platelets co-encapsulated in the hydrogel (T-Platelet@gel), P-aPDL1-loaded gel (P-aPDL1@gel), CAR-T cells and isotype antibody-conjugated platelet encapsulated in the hydrogel (CAR-T-P-isotype antibody@gel), free CAR-T cells, free CAR-T cells and P-aPDL1, CAR-T@gel, CAR-T-loaded gel and P-aPDL1(CAR-T@gel+P-aPDL1), CAR-T-loaded gel and systemic injection of aPDL1 (CAR-T@gel+Systemic aPDL1), and CAR-T-P-aPDL1@gel. Tumor recurrence was monitored by measuring tumor bioluminescence signals. As shown in FIGS. 3A, 3B and FIGS. 21A-21C, saline and T-Platelet@gel groups showed no therapeutic effects. While mice treated with free CAR-T cells with or without P-aPDL1 only achieved moderate anti-tumor effects, mice treated with CAR-T@gel+P-aPDL1 (therapeutic hydrogel material 10) the showed the most prominent protection from tumor recurrence. CAR-T-P-aPDL1@gel displayed higher anti-tumor activity even compared with CAR-T@gel+Systemic aPDL1. Quantitative bioluminescence intensity of the tumors treated with CAR-T-P-aPDL1@gel at week 3 was 6.4-fold lower than CAR-T@gel+P-aPDL1 group and over 60-fold lower than other treatment groups (FIG. 3C). Direct measurement of the tumor volume, showed that mice treated with CAR-T-P-aPDL1@gel at week 3 displayed significantly smaller tumors as compared to other treatment groups (FIGS. 3D, 3E).

### In vivo CAR-T proliferation and abscopal treatment efficacy

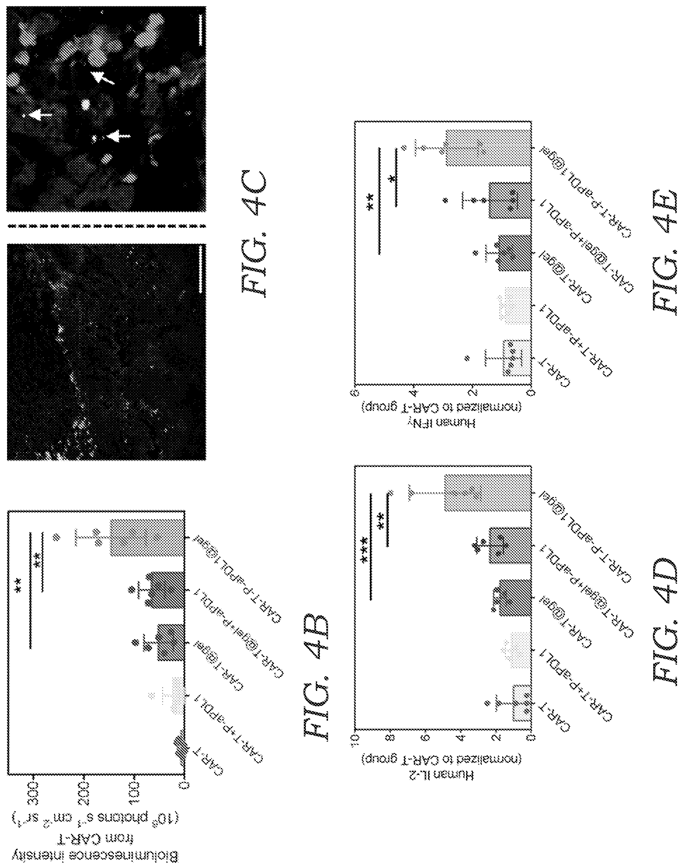

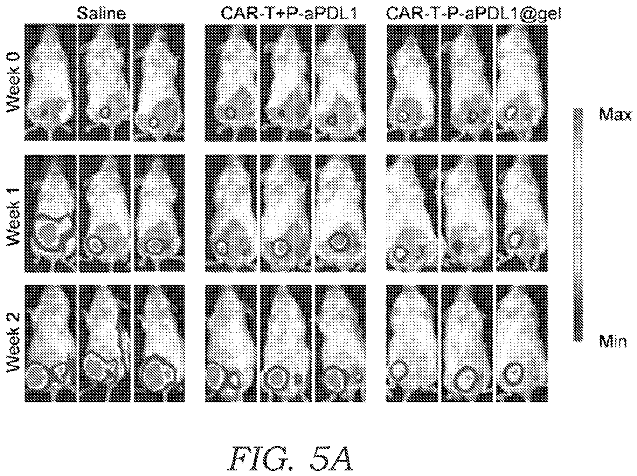

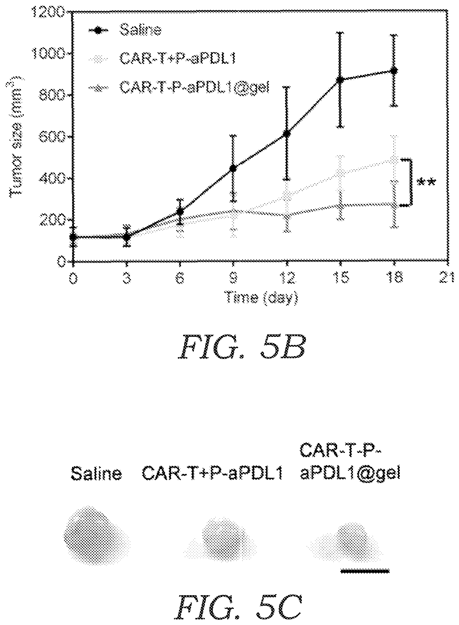

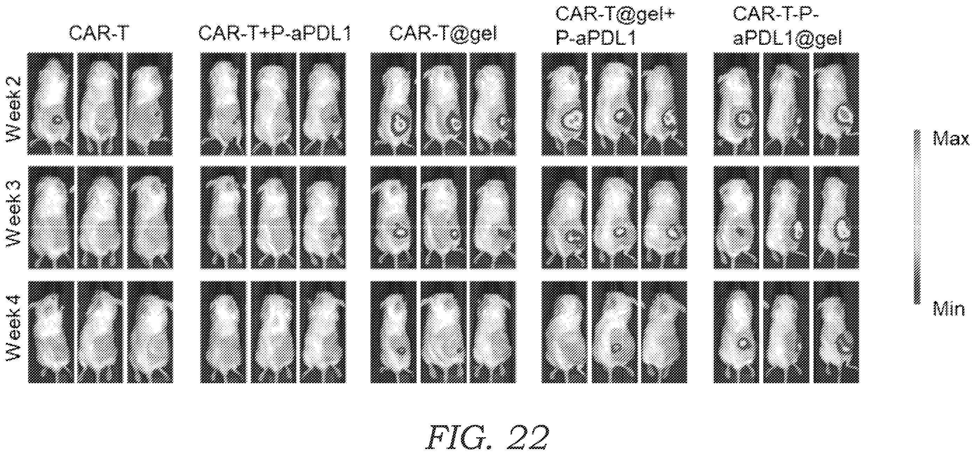

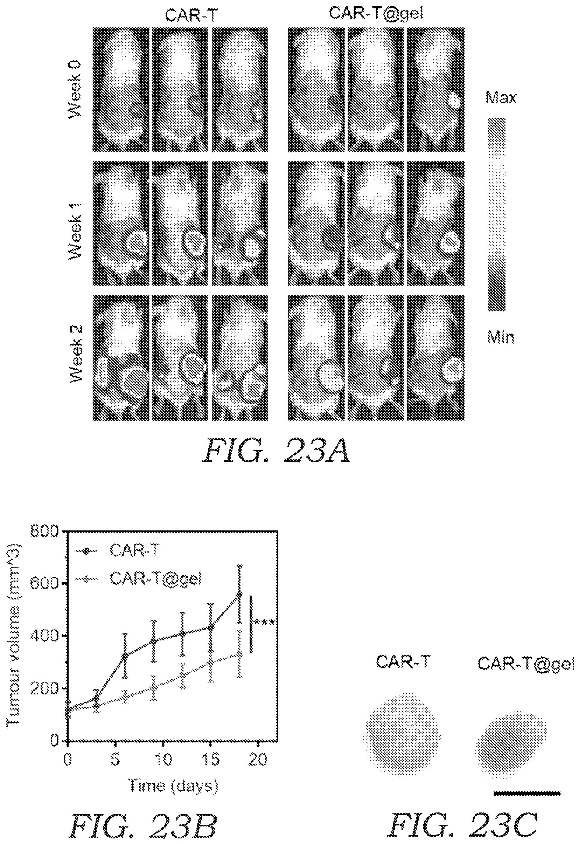

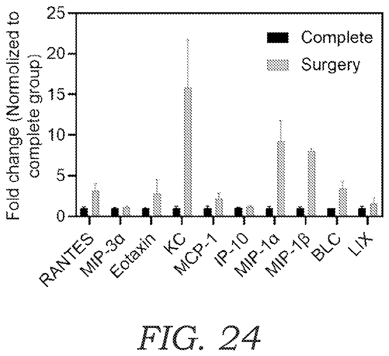

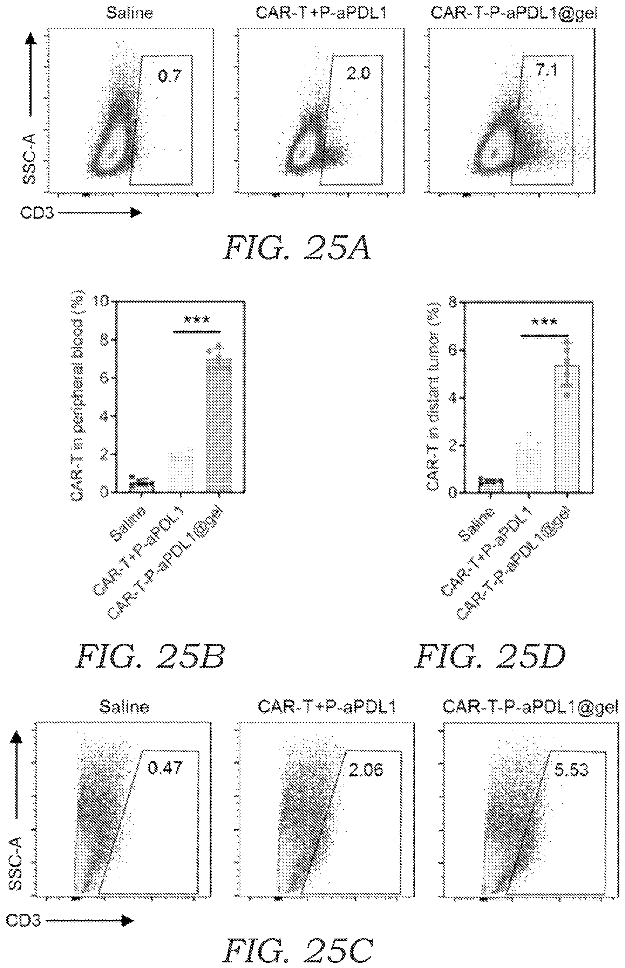

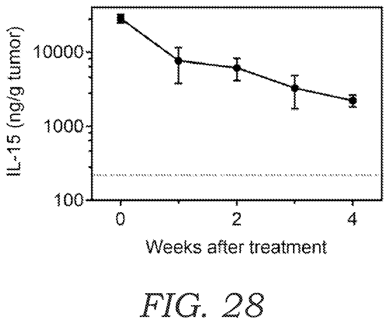

To investigate the underlying mechanism of superior anti-tumor efficacy of the therapeutic hydrogel material 10 (i.e., CAR-T-P-aPDL1@gel), CAR-T cells 12 were labeled with luciferase to track their persistence *in vivo* (FIG. 12). As shown in the FIG. 4A, bioluminescence of free CAR-T cells rapidly disappeared, while the bioluminescence of CAR-T cells 12 loaded in the hydrogel 10 persisted as demonstrated by over 3-fold increase when compared to other treatments (FIG. 4B). The long-term persistence of CAR-T cells 12 were further monitored by *in vivo* imaging. The therapeutic hydrogel material 10 (CAR-T-P-aPDL1@gel) showed detectable signal up to week 4, while all other treatment groups displayed negligible signals (FIG. 22). As shown in the FIG. 4C, T cell bioluminescence correlated with higher detection of tumor infiltrating CAR-T cells 12. In addition, PMPs were detected within the tumor indicating the undergoing activation of transferred platelets due to the local inflammation (FIG. 4C). Cytokine levels within the tumor were also examined to further demonstrate that CAR-T cells 12 released IFNy and IL-2 locally upon antigen encountering (FIGS. 4D, 4E). Moreover, IL-15 was detectable within the tumor throughout the time-course treatment and was significantly higher than untreated tumors (FIG. 28). To investigate if CAR-T cells 12 locally delivered at the tumor site via CAR-T-P-aPDL1@gel cause systemic tumor protection, a double tumor model was established in which tumor cells were implanted in both flanks in NSG mice. When the tumor size reached the size of ~100 mm³, surgery was performed on the tumor located on right side and hydrogel 10 was implanted. As shown in FIG. 5A and FIGS. 23A-23C, CAR-T-P-aPDL1@gel displayed an abscopal effect causing inhibition of the tumor growth in the contralateral site (FIGS. 5B, 5C and FIGS. 23B, 23C). To explain the underlying mechanism of the abscopal effects, the chemokine gradients within the tumor were first analyzed before and after surgical resection. The majority of chemokines included in the chemokine array used showed significantly higher levels after surgery (FIG. 24). Moreover, CAR-T cells 12 delivered via CAR-T-P-aPDL1@gel (therapeutic hydrogel material 10) in the resection cavity reached the blood stream because CAR-T cells 12 were also detectable in peripheral blood (FIGS. 25A, 25B) and at the distant tumor site (FIGS. 25C-25E). Collectively, the combination of a favorable chemokine milieu within the tumor created by the surgical resection and the recirculation of CAR-T cells 12 support the control of tumor growth at distant sites by locally delivered CAR-T cells 12.

### Discussion

A biocompatible therapeutic hydrogel material 10 is disclosed that can deliver intratumorally CAR-T cells 12 and enhance T cell persistence as compared to free injected CAR-T cells. Furthermore, as demonstrated herein, anti-PDL1 blocking antibody conjugated platelets 14 can act as bioresponsive cells and release intratumorally PDL1 blockades upon activation. This in turn sustains CAR-T cell antitumor activity protecting them from exhaustion.

Resection of the tumor mass is frequently only partially effective and adjuvant chemotherapy and radiotherapy are used in the attempt to eradicate the tumor. The development of on-site drug delivery may reveal a more specific and effective approach to prevent tumor recurrence. The on-site drug delivery platform disclosed herein can be adapted to cellular therapies and in particular to adoptive T cell therapies. Preclinical and clinical studies have shown the feasibility of intratumor delivery of CAR-T cells with the intent to concentrate T cells at the tumor site overcoming the challenge of T cell biodistribution within the tumor and the potential side effects encountered when CAR-T cells are targeting antigens shared by normal tissues. Here it was found that the hyaluronic acid-based hydrogel material 10 preserves fully functional CAR-T cells 12 and also allows the encapsulation of nanoparticles 18 carrying growth factors for CAR-T cells, which represents another advantageous layer to enhance their antitumor activity.

In addition to highly functional effector T cells concentrated at the tumor site, a curative immune response should also reverse the immunosuppressive tumor microenvironment. It was found that the hyaluronic acid-based hydrogel material 10 is a highly flexible delivery system allowing encapsulation of more than one functional cell type. Specifically, a strategy was developed that involves the local delivery of platelets 14 in combination with CAR-T cells 12. Platelets 14 can be decorated with antibodies allowing for example the local biodistribution of a checkpoint blockade. Furthermore, platelets 14 are responsive to inflammation secondary to the wound healing process and form microplatelets that promote effective biodistribution of the loaded checkpoint blockade to tumor cells. Finally, activated platelets are source of ligands, such as CD40L, and chemokines that boost CAR-T immunity and recruit other immune cells further amplifying the antitumor effects. Collectively, these findings substantiated the rationale of combining platelets 14 and CAR-T cells 12 as antitumor agents.

The CAR-T cells 12 and platelets 14 can be combined and delivered making use of an optimized hyaluronic-acid hydrogel formulation that can be implanted directly into the tumor bed upon surgical resection. The therapeutic hydrogel material 10 supports T cell viability by introducing nanoparticles 18 delivering IL15, while platelets 14 delivering PDL1 synergize in protecting T cell from exhaustion. As a result, the formulated therapeutic hydrogel material 10 remarkably controlled local tumor recurrence and triggered an abscopal effect inhibiting distant tumor growth. While the therapeutic hydrogel material 10 was used to treat melanoma, it should be appreciated that the therapeutic hydrogel material 10 may be used with the treatment of other cancers such as pancreatic cancer and breast cancer.

The therapeutic hydrogel material 10 for post-surgical immunotherapy is cost effective as compared to the systemic administration of multiple single agents. Furthermore, the proposed hydrogel reservoir can be easily manipulated to incorporate other therapeutic bio-particulates to form a local "immune cell factory" that eliminate residual tumor cells.

### Methods

### Cell lines and cells

The human melanoma WM115 cells were tagged with the fusion protein eGFP-firefly-luciferase. Human T cells engineered with CSPG4. CAR were generated as previously described in Pellegatta, S. et al. Constitutive and TNFα-inducible expression of chondroitin sulfate proteoglycan 4 in glioblastoma and neurospheres: Implications for CAR-T cell therapy. Sci. Transl. Med. 10, eaao2731 (2018). Here, experiments used a CSPG4-specific CAR that encodes the CD28 endodomains with enhanced functionality. WM115 cells were maintained in Dulbecco's modified Eagle's medium (Gibco; Invitrogen) supplemented with 10% fetal bovine serum (Invitrogen), 100 U/ml penicillin (Invitrogen) and 100 U/ml¹ streptomycin (Invitrogen). CAR-T cells 12 were cultured in in complete medium containing 45% RPMI 1640 and 45% Click's medium (Irvine Scientific) with 10% FBS (HyClone), 2 mmol/L GlutaMAX, human recombinant interleukin-7 (5 ng/mL, Pepro Tech Inc) and human recombinant interleukin-15 (10 ng/ml, Pepro Tech Inc). Cells were cultured in an incubator (Thermo Fisher Scientific) at 37 °C under an atmosphere of 5% CO₂ and 90% relative humidity. The cells were sub-cultivated approximately every 2 - 3 d at 80% confluence at a split ratio of 1:3.

### Fabrication and characterization of IL-15 nanoparticle

Ten mg PLGA was dissolved in 2 ml CH₂Cl₂ to prepare oil phase and 0.4 ml 1% w/v PVA solution containing 50 µg/ml IL-15 was prepared for water phase. The CH₂Cl₂ solution was slowly added to PVA solution by sonication. Thereafter, 10 ml 2% w/v PVA solution was added to the previous emulsion mixture and sonicated to obtain double emulsion solution. IL-15 nanoparticle 18 solution was applied to the rotary evaporator for dichloromethane evaporation. Afterward, the emulsion was centrifuged to collect IL-15-NPs 18. Hydrodynamic size of IL-15-NPs 18 was studied by Zetasizer (Nano ZS, Malvern). The morphology of IL-15-NPs 18 was characterized by Transmission Electron Microscopy (TEM) (JEM-2000FX, Hitachi) after being stained with 1% (w:v) phosphotungstic acid. The amount of IL-15 loaded in the hydrogel was 1 µg.

To study *in vitro* the release of IL-15 from IL-15-NPs 19, 1 ml IL-15-NPs 19 were incubated with PBS of pH 7.4 in a shaker (New Brunswick Scientific). At pre-determined time points, IL-15 released out was determined using a specific ELISA kit (Abcam, USA).

### Preparation of platelets loaded with the aPDL1 antibody

Human platelet concentrate was purchased from Zen-Bio, Inc (North Carolina). The human platelet concentrate was centrifuged for 20 minutes at 100 g and then at 800g for 20 minutes to collect platelet. The platelet pellet was collected and resuspended in phosphate buffered saline (PBS) containing 1 µM PGE1 for further use. Platelets were counted using the hemocytometer under a microscope. The platelet solution was centrifuged at 800 g for 20 minutes and washed with PBS to remove the PGE1 for the platelet activation study.

The conjugation of the aPDL1 on the surface of platelets was performed as previously described in Hu, Q. et al. Conjugation of hematopoietic stem cells and platelets decorated with anti-PD-1 antibodies augments anti-leukemia efficacy. Nature Biomedical Engineering 2, 831 (2018). Briefly, platelets were incubated with Traut's reagent to generate thiol groups. The aPDL1 (e.g., BioLegend (catalogue number 114114, clone: RMP1-14) was reacted with Sulfo-SMCC at a molar ratio of 1:1.2 for 2 h at 4 °C. The excess SMCC linker was discarded by centrifugation in an ultrafiltration tube (MW cut-off, 3 kDa). The SMCC-aPDL1 was added to the platelets and stirred for 2 hours at room temperature. aPDL1 was purified by repeatedly washed with PBS and centrifuged at 800 g. To determine the amount of conjugated aPDL1 on the surface of platelets 14, aPDL1 was lysed with ultrasonication in 0.1% Triton buffer and the released aPDL1 was measured using a specific ELISA kit (Human IgG Total ELISA Kit, abcam). The release profile of aPDL1 was investigated with addition of thrombin (0.5 U ml⁻¹). Briefly, 1 × 10⁸ P-aPDL1 were added with thrombin and maintained at 37 °C without stirring. At pre-determined time points, the released aPDL1 in the supernatant was measured by ELISA kit after centrifugation at 800 g for 20 minutes.

To demonstrate the existence of the aPDL1 on the surface of platelets 14, aPDL1 was labeled with fluorescein isothiocyanate-NHS (FITC) before reacted with rhodamine B-stained platelets. Afterward, the fluorophores-labeled P-aPDL1 was visualized by confocal microscope. Furthermore, the binding of aPDL1 to platelets 14 was also analyzed by flow cytometry after staining with a FITC-labeled anti-human IgG antibody. The functionality of P-aPDL1 was examined by collagen-binding experiments. Briefly, human collagen type IV (2 mg/ml) was added to a confocal dish and maintained at 4 °C for overnight. Afterward, the confocal dish was washed with PBS and further blocked by 2% bovine serum albumin for 2 h. The confocal dish with collagen pre-treatment was set as control. 1×10⁶ P-aPDL1 or naive platelets stained with Wheat germ agglutinin Alexa Fluor 594 were added to the dishes. One minute later, the dishes were washed with PBS and subjected to confocal microscope for imaging.

### Preparation of cell-loaded HA hydrogel

The hyaluronic acid was modified with the methacrylic anhydride (MA) to form double bond as previously described in Jiang, T., Mo, R., Bellotti, A., Zhou, J. & Gu, Z. Gel-Liposome-Mediated Co-Delivery of Anticancer Membrane-Associated Proteins and Small-Molecule Drugs for Enhanced Therapeutic Efficacy. Adv. Funct. Mater. 24, 2295-2304 (2014). To form HA hydrogel, 400 µl HA solution (4% w/v) together with *N,N*-methylenebisacrylamide (MBA) (MBA:HA, 1:5, w:w) and a photo-initiator Irgacure^{™} 2959 (0.1%, w:v) were added into a 48-well plate. After radical polymerization *via* UV radiation for 25 minutes using a BlueWave 75 UV Curing Spot Lamp (DYMAX), the obtained hydrogel was subjected to lyophilizer for 48 hours. To load the cells 12 into the hydrogel, the lyophilized HA-hydrogel was placed on ice and 1 × 10⁶ CAR-T cells 12, and 1 × 10⁷ platelets 14 loaded with aPDL1 were seeded. Thereafter, the hydrogel 10 was maintained in ice for 15 - 20 minutes to ensure sufficient inclusion of cells 12.

### Characterization of the cells loaded into the hydrogel

To visualize the distribution of cells in the hydrogel, CAR-T cells 12 were labeled with WGA-Alexa Fluor 488 and Hoechst 33452. Platelets were labeled with WGA-Alexa Fluor 594. CAR-T cells 12 (1×10⁶ cells) and platelets 14 (1×10⁷ cells) were seeded into the hydrogel 10, embedded with OCT and immerged in liquid nitrogen. Slides obtained from frozen material were analyzed by confocal microscope. Morphology was characterized by cryo-SEM (JEOL 7600F, Gatan Alto). Functionality and viability of CAR-T cells 12 in the hydrogel 10 was examined after degradation of the HA-hydrogel 10. Briefly, the hydrogel material 10 containing 1×10⁷ CAR-T cells 12 was placed in a cell strainer with 40 µm pore size and embedded in a 6-well plate. After 48 hours, IL-2 and IFNy released in the medium were measured by ELISA kit (Abcam). For CAR-T cell viability, the hydrogel 10 was treated with HAase (1 mg/ml) for 1 hour to digest the HA matrix. CAR-T cells 12 released were collected by centrifugation at 500 g for 5 minutes and stained for live/dead cell assay kit (Thermos fisher) and imaged by fluorescence microscope.

To measure the hydrogel loading efficiency, CAR-T cells 12 and IL-15 with Cy5.5 fluorophore were loaded in the hydrogels in a 48-well plate. After 15 min, hydrogels with the CAR-T cells 12 (2×10⁶) and IL-15-NP 18 were transferred to another well. The fluorescence intensity in the original wells was measured by imaging to evaluate cells 12/nanoparticles 18 leaked out from the hydrogel 10. To study the degradation *in vivo* of the HA-hydrogel material 10, the HA-hydrogel labeled with Cy5.5 was implanted in the NSG mouse subcutaneously. Fluorescence imaging was recorded by an IVIS Spectrum imaging system (Perkin Elmer).

### Release and mobility of the cells within the hydrogel

Hydrogel material 10 containing 1×10⁷ CAR-T cells 12 and 1×10⁷ platelets 14 was placed a cell strainer with 40 µm pore size. The strainer was embedded in a 6-well plate with 5 ml of medium without any cytokine. At pre-determined time points, CAR-T cells 12 and platelets 14 were counted using the trypan blue exclusion. To evaluate the release of CAR-T cells 12 *in vivo,* CAR-T cells 12 were stained with Celltracker deep red for 30 mins. The hydrogel loaded with 2×10⁶ fluorophore-labeled CAR-T cells 12 was implanted into the tumor resection cavity. The hydrogel material 12 was then removed every day for a week, and CAR-T cells 12 released in the resection cavity were quantified by *in vivo* imaging. The analysis of signals was performed on by Living Image Software version 4.3.1.

Cell mobility within the hydrogel was characterized by labelling CAR-T cells 12 with CellTracker Orange CMRA and platelets 14 with CellTracker Green CMFDA (Thermo Fisher). Cells was imaged by confocal microscope equipped with a humidified environmental chamber (37 °C, 5% CO2), and images were recorded every 30 seconds for 30 minutes. The cell track was analyzed by Imaris imaging analysis software.

### CAR-T and tumor cells co-culture study

To killing effects of CAR-T cells 12, the T cells in various formulations, including T cell loaded in the hydrogel (T@gel, T cell number 3.3×10⁵), T cells and platelets loaded in the hydrogel (T-Platelet@gel, T cells 3.3×10⁵, platelets 1×10⁷), CAR-T cells 12 loaded in the hydrogel (CAR-T@gel, CAR-T cell number 3.3×10⁵), and CAR-T cells 12 and platelets 14 loaded in the hydrogel 10 (CAR-T-P-aPDL1@gel, CAR-T cell number 3.3×10⁵, platelet number 1×10⁷, aPDL1 1 µg), were placed in a strainer with 40 µm pore size and embedded in a 6-well plate seeded with 1 × 10⁶ GFP-labeled WM-115 cells. After 72 hours, cells were trypsinized and collected for flow cytometry analysis. IL-2 and IFNy released in the media were measured by ELISA kits.

Proliferation of CAR-T cells 12 was measured using the carboxyfluorescein succinimidyl ester (CFSE) staining assay. Briefly, T cells were stained with CFSE (5 µM) according to the manufacturer's instructions, seeded in the hydrogel and co-cultured with WM115 melanoma cells. After 96 hours, cells were collected after trypsinization, stained with the anti-CD3 antibody and analysis by flow cytometry. PDL1 expression of WM115 after co-cultured with CAR-T cells 12 was analyzed by flow cytometry. All the *in vitro* CAR-T cell studies were performed without addition of the IL-15-NPs.

### In vivo anti-tumor efficacy

The NOD.Cg-Prkdc^{scid} Il2rg^{tm1Wjl}/SzJ (NSG) mice (female, 6 - 8 weeks) were purchased from Jackson laboratory. All the animal studies strictly followed the animal protocol approved by the Institutional Animal Care and Use Committee at the University of North Carolina at Chapel Hill and North Carolina State University. To establish *in vivo* human WM115 melanoma model, NSG mice were injected with 5 × 10⁶ WM115 cells subcutaneously. After two weeks, the tumors were partially removed and embedded in OCT to obtain frozen sections. Slides of tumor sections were stained with the anti-PDL1 and anti-CSPG4 antibodies and Hoechst 33342, and then subjected to confocal microscope imaging. Control WM115 tumors and CAR-T@gel- (CAR-T cells number: 2×10⁶) treated tumors were digested by collagenase, mechanically disrupted to single cell suspension, stained with BV421-anti human PDL1 (Clone: 29E.2A3) antibody and analyzed by flow cytometry. An irrelevant antibody was used as the isotype control.

For *in vivo* anti-tumor studies, NSG mice were injected with 5 × 10⁶ luciferase-labeled WM115 cells subcutaneously. When the tumor size reached to ~150 mm³, tumors were partially removed leaving ~5% of the tumor mass. Resection cavities were filled with various formulations that include saline, T-Platelet@gel, P-aPDL1@gel, CAR-T, CAR-T-P-isotype antibody@gel, CAR-T+P-aPDL1, CAR-T@gel, CAR-T@gel+P-aPDL1, CAR-T@gel+Systemic aPDL1, CAR-T-P-aPDL1@gel (2×10⁶ CAR-T cells, 1×10⁷ platelets, aPDL1, 1 µg). The dose and frequency for systemic injection of aPDL1 were 10 µg every day for 3 days *via* tail vein injection. In all experimental groups IL-15-NP 18 (IL-15: 1 µg) were included. To visualize the luciferase signal, mice were injected intraperitoneally with D-Luciferin at a dose of 150 mg per kg in 100 µl PBS. Mice were imaged after 5 minutes with 1 s of acquisition time. Mice were imaged at 1, 2 and 3 weeks after hydrogel implantation. Bioluminescence signals were analyzed by Living Image Software version 4.3.1 (PerkinElmer). After 3 weeks, tumors were removed and imaged. Tumor volume were recorded by a digital caliper and calculated according to the formula: length × width² ×0.5.

To investigate the abscopal effects of CAR-T-PaPDL1@gel (i.e., therapeutic hydrogel material 10), the double tumor-bearing NSG mouse model was built by injected 5×10⁶ luciferase-labeled WM115 cells at the abdomen site subcutaneously. When tumor sizes reached 100 mm³, the tumor on the right size was surgically removed (leaving ~5% residual tumor) and resection cavities filled with different formulations: saline, CAR-T, CAR-T@gel, CAR-T + P-aPDL1, and CAR-T-P-aPDL1@gel (CAR-T, 2×10⁶, platelet, 1×10⁷, aPDL1, 1 µg). In all experimental groups IL-15-NP 18 (IL-15: 1 µg) were included. Mice were imaged on week 1 and 2 to monitor tumor recurrence on the surgical side, while the growth of the untouched tumors on left side was monitored with a digital caliper. Mice were also bled to evaluate the redistribution of CAR-T cells 12 in the peripheral blood. Hundred µl blood sample was collected and red blood cells were lysed. CAR-T cells 12 in the peripheral blood were detected using FITC-labeled anti-human CD45 and APC-labeled anti-human CD3 antibodies and analyzed by flow cytometry. To analyze the presence of CAR-T cells 12 in the distant tumor, tumors were removed, digested with collagenase for 30 min, mechanically disrupted and filtered through cell strained with pore size of 40 µm. Cell suspensions were stained with APC-labeled anti-human CD3 antibody and analyzed by flow cytometry. Tumors were also embedded with OCT and immerged in liquid nitrogen. Slides obtained from frozen material were analyzed by confocal microscope after staining with Hoechst 33342, PE labeled anti-human CD4 and FITC-labeled anti-human CD8 antibodies.

### In vivo CAR-T cells expansion

The resection tumor model was established as previously described. After surgery, the resection cavity was treated with CAR-T, CAR-T+P-aPDL1, CAR-T@gel, CAR-T@gel+P-aPDL1, CAR-T-P-aPDL1@gel (CAR-T cell number, 2×10⁶, Platelet number 1×10⁷, aPDL1, 1 µg). In all experimental groups IL-15-NP 18 (IL-15: 1 µg) were included. CAR-T cells 12 were co-transduced with a vector encoding the fusion protein GFP-Firefly luciferase for *in vivo* imaging. Bioluminescence signals were recorded on day 1 after implantation and then at day 4, 7, 14, 21 and 28. The analysis of signals was performed on by Living Image Software version 4.3.1.

To investigate the tumor distribution of both CAR-T cells 12 and platelets 14, CAR-T cells 12 were stained with CellTracker Orange and platelets 14 were labeled with WGA-Alexa Fluor 488. After tumor removal, the CAR-T-P-aPDL1@gel (therapeutic hydrogel material 10) was implanted and the tumor tissue was taken out after 72 hours for frozen section. After staining with Hoechst 33254, the slide was subjected to confocal microscope for observation. Cytokine levels in the tumors were measured by LEGENDplex human and mouse cytokine detection kit. Tumors were harvested 7 days after treatment, weighted and mechanically disrupted. After homogenized in cold PBS, the cell mixture was filtered through the cell strainer, and after centrifugation analyzed by flow cytometry using the software provided by manufacturer.

To investigate the chemokine levels in the WM1115 tumor after surgery, when the tumor size reached to ~150 mm³, tumors were partially removed leaving ~5% of the tumor mass. After 2 days, the surgical tumors and complete tumor were harvested and mechanically disrupted. After homogenized in cold PBS, the cell mixture was filtered through the cell strainer, and after centrifugation analyzed by flow cytometry using the software provided by manufacturer. The chemokine was measured by LEGENDplex human chemokine detection kit.

### Statistics

All results are presented as means ± s.d. Statistical analysis was evaluated using GraphPad Prism (7.0). One-way analysis of variance (ANOVA) followed by Tukey's post *hoc* test was performed for multiple groups analyses and un-paired Student *t*-test was performed for two groups analysis. The differences between experimental groups and control groups were considered statistically significant at *P* < *0.05. *P* < 0.05; ***P* < 0.01; and ****P* < 0.001.

## Claims

1. A therapeutic hydrogel material for the delivery of chimeric antigen receptor (CAR)-engineered T cells (CAR-T cells) to mammalian tissue comprising:
a biocompatible hydrogel material containing therein CAR-T cells targeting human chondroitin sulphate proteoglycan 4 (CSPG4) and human platelets conjugated to anti-PDL1 blocking antibody (aPDL1).

2. The therapeutic hydrogel material of claim 1, wherein the hydrogel material further contains therein nanoparticles loaded with one or more cytokines.

3. The therapeutic hydrogel material of claim 2, wherein the one or more cytokines comprises interleukin 15 (IL-15) or interleukin 7 (IL-7).

4. The therapeutic hydrogel material of any of claims 1-3, wherein the biocompatible hydrogel material is biodegradable.

5. The therapeutic hydrogel material of any of claims 1-4, wherein the biocompatible hydrogel material is a hyaluronic acid-based hydrogel.

6. The therapeutic hydrogel material of claim 5, wherein the hyaluronic acid-based hydrogel is modified with methacrylic anhydride (MA).

7. The therapeutic hydrogel material of any of claims 1-4, wherein the biocompatible hydrogel material comprises a polysaccharide, acrylic polymer, or protein.

8. A method of making a therapeutic hydrogel material for the delivery of chimeric antigen receptor (CAR)-engineered T cells (CAR-T cells) to mammalian tissue comprising:
forming a crosslinked biocompatible hydrogel material;
lyophilizing the crosslinked biocompatible hydrogel material; and
loading one or more solutions of CAR-T cells targeting human chondroitin sulphate proteoglycan 4 (CSPG4) and human platelets conjugated to anti-PDL1 blocking antibody (aPDL1) into the biocompatible hydrogel material.

9. The method of claim 8, further comprising loading nanoparticles containing one or more cytokines therein into the biocompatible hydrogel material.

10. The method of claim 9, wherein the nanoparticles contain interleukin 15 (IL-15) or interleukin 7 (IL-7).

11. The method of any of claims 8-10, wherein the biocompatible hydrogel material is biodegradable.

12. The method of any of claims 8-11, wherein the biocompatible hydrogel material is a hyaluronic acid-based hydrogel.

13. The method of claim 12, wherein the hyaluronic acid-based hydrogel is modified with methacrylic anhydride (MA).

14. The method of any of claims 8-11, wherein the biocompatible hydrogel material comprises a polysaccharide, acrylic polymer, or protein.

## Patentansprüche

1. Ein therapeutisches Hydrogelmaterial für die Abgabe von chimären Antigenrezeptor (CAR)-modifizierten T-Zellen (CAR-T-Zellen) an das Gewebe eines Säugetiers, umfassend:
ein biokompatibles Hydrogelmaterial, das CAR-T-Zellen enthält die auf menschliches Chondroitinsulfat-Proteoglycan 4 (CSPG4) abzielen, und menschliche Thrombozyten, die mit anti-PDL1 blockierenden Antikörpern (aPDL1) konjugiert sind.

2. Das therapeutische Hydrogelmaterial nach Anspruch 1, wobei das Hydrogelmaterial weiter darin Nanopartikel enthält, die mit einem oder mehreren Zytokinen beladen sind.

3. Das therapeutische Hydrogelmaterial nach Anspruch 2, wobei das eine oder die mehreren Zytokine Interleukin 15 (IL-15) oder Interleukin 7 (IL-7) umfassen.

4. Das therapeutische Hydrogelmaterial nach einem der Ansprüche 1-3, wobei das biokompatible Hydrogelmaterial biologisch abbaubar ist.

5. Das therapeutische Hydrogelmaterial nach einem der Ansprüche 1-4, wobei das biokompatible Hydrogelmaterial ein hyaluronsäure-basiertes Hydrogel ist.

6. Das therapeutische Hydrogelmaterial nach Anspruch 5, wobei das hyaluronsäure-basierte Hydrogel mit Methacrylsäureanhydrid (MA) modifiziert ist.

7. Das therapeutische Hydrogelmaterial nach einem der Ansprüche 1-4, wobei das biokompatible Hydrogelmaterial ein Polysaccharid, Acrylpolymer oder Protein umfasst.

8. Ein Verfahren zur Herstellung eines therapeutischen Hydrogelmaterials für die Abgabe von chimären Antigenrezeptor (CAR)-modifizierten T-Zellen (CAR-T-Zellen) an das Gewebe eines Säugetiers, umfassend:
Bildung eines vernetzten biokompatiblen Hydrogelmaterials;
Lyophilisierung des vernetzten biokompatiblen Hydrogelmaterials; und
Laden einer oder mehrerer Lösungen von CAR-T-Zellen, die auf menschliches Chondroitinsulfat-Proteoglycan 4 (CSPG4) abzielen, und menschliche Thrombozyten, die mit anti-PDL1 blockierenden Antikörpern (aPDL1) konjugiert sind, in das biokompatible Hydrogelmaterial.

9. Das Verfahren nach Anspruch 8, weiter umfassend das Laden von Nanopartikeln, die eines oder mehrere Zytokine enthalten, in das biokompatible Hydrogelmaterial.

10. Das Verfahren nach Anspruch 9, wobei die Nanopartikel Interleukin 15 (IL-15) oder Interleukin 7 (IL-7) enthalten.

11. Das Verfahren nach einem der Ansprüche 8-10, wobei das biokompatible Hydrogelmaterial biologisch abbaubar ist.

12. Das Verfahren nach einem der Ansprüche 8-11, wobei das biokompatible Hydrogelmaterial ein hyaluronsäure-basiertes Hydrogel ist.

13. Das Verfahren nach Anspruch 12, wobei das hyaluronsäure-basierte Hydrogel mit Methacrylsäureanhydrid (MA) modifiziert ist.

14. Das Verfahren nach einem der Ansprüche 8-11, wobei das biokompatible Hydrogelmaterial ein Polysaccharid, Acrylpolymer oder Protein umfasst.

## Revendications

1. Un matériau hydrogel thérapeutique pour la délivrance de cellules T modifiées avec des récepteurs d'antigène chimérique (cellules CAR-T) dans les tissus mammifères comprenant :
un matériau hydrogel biocompatible contenant des cellules CAR-T ciblant le protéoglycane de sulfate de chondroïtine 4 (CSPG4) humain et des plaquettes humaines conjuguées à un anticorps bloquant anti-PDL1 (aPDL1).

2. Le matériau hydrogel thérapeutique selon la revendication 1, dans lequel le matériau hydrogel contient en outre des nanoparticules chargées avec une ou plusieurs cytokines.

3. Le matériau hydrogel thérapeutique selon la revendication 2, dans lequel les une ou plusieurs cytokines comprennent l'interleukine 15 (IL-15) ou l'interleukine 7 (IL-7).

4. Le matériau hydrogel thérapeutique selon l'une quelconque des revendications 1 à 3, dans lequel le matériau hydrogel biocompatible est biodégradable.

5. Le matériau hydrogel thérapeutique selon l'une quelconque des revendications 1 à 4, dans lequel le matériau hydrogel biocompatible est un hydrogel à base d'acide hyaluronique.

6. Le matériau hydrogel thérapeutique selon la revendication 5, dans lequel l'hydrogel à base d'acide hyaluronique est modifié avec l'anhydride méthacrylique (MA).

7. Le matériau hydrogel thérapeutique selon l'une quelconque des revendications 1 à 4, dans lequel le matériau hydrogel biocompatible comprend un polysaccharide, un polymère acrylique, ou une protéine.

8. Un procédé de fabrication d'un matériau hydrogel thérapeutique pour la délivrance de cellules T modifiées avec des récepteurs d'antigène chimérique (cellules CAR-T) dans les tissus mammifères comprenant :
former un matériau hydrogel biocompatible réticulé ;
lyophiliser le matériau hydrogel biocompatible réticulé ; et
charger une ou plusieurs solutions de cellules CAR-T ciblant le protéoglycane de sulfate de chondroïtine 4 (CSPG4) humain et des plaquettes humaines conjuguées à un anticorps bloquant anti-PDL1 (aPDL1) dans le matériau hydrogel biocompatible.

9. Le procédé selon la revendication 8, comprenant en outre le chargement de nanoparticules contenant une ou plusieurs cytokines dans le matériau hydrogel biocompatible.

10. Le procédé selon la revendication 9, dans lequel les nanoparticules contiennent l'interleukine 15 (IL-15) ou l'interleukine 7 (IL-7).

11. Le procédé selon l'une quelconque des revendications 8 à 10, dans lequel le matériau hydrogel biocompatible est biodégradable.

12. Le procédé selon l'une quelconque des revendications 8 à 11, dans lequel le matériau hydrogel biocompatible est un hydrogel à base d'acide hyaluronique.

13. Le procédé selon la revendication 12, dans lequel l'hydrogel à base d'acide hyaluronique est modifié avec l'anhydride méthacrylique (MA).

14. Le procédé selon l'une quelconque des revendications 8 à 11, dans lequel le matériau hydrogel biocompatible comprend un polysaccharide, un polymère acrylique, ou une protéine.
